(19) Europäisches Patentamt / European Patent Office / Office européen des brevets

(11) **EP 4 326 353 B1**

(12) **EUROPEAN PATENT SPECIFICATION**

(45) Date of publication and mention
of the grant of the patent:
**18.06.2025 Bulletin 2025/25**

(21) Application number: **22721125.7**

(22) Date of filing: **19.04.2022**

(51) International Patent Classification (IPC):
*A61L 15/44* (2006.01)   *A61L 26/00* (2006.01)
*A61L 31/16* (2006.01)   *A01N 59/20* (2006.01)
*A01N 59/16* (2006.01)   *A61P 31/04* (2006.01)
*A61P 31/12* (2006.01)

(52) Cooperative Patent Classification (CPC):
(C-Sets available)
**A61L 15/44; A01N 25/34; A01N 59/16;
A61L 26/0066; A61L 31/16; A61P 31/04;
A61P 31/12;** A61L 2300/404          (Cont.)

(86) International application number:
**PCT/IB2022/053656**

(87) International publication number:
**WO 2022/224142 (27.10.2022 Gazette 2022/43)**

(54) **ENHANCED ANTIMICROBIAL EFFICACY (SYNERGY) OF SILVER AND COPPER COMPOUNDS AND MEDICAL USE OF THEIR COMBINATIONS**

ERHÖHTE ANTIMIKROBIELLE WIRKSAMKEIT (SYNERGIE) VON SILBER- UND KUPFERVERBINDUNGEN UND MEDIZINISCHE VERWENDUNG IHRER KOMBINATIONEN

EFFICACITÉ ANTIMICROBIENNE AMÉLIORÉE (SYNERGIE) DE COMPOSÉS D'ARGENT ET DE CUIVRE ET UTILISATION MÉDICALE DE LEURS COMBINAISONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2021   US 202163176617 P
22.12.2021   US 202163292472 P**

(43) Date of publication of application:
**28.02.2024 Bulletin 2024/09**

(73) Proprietor: **Nanordica Medical Oü
10134 Tallinn (EE)**

(72) Inventors:
• **BONDARENKO, Olesja
13420 Tallinn (EE)**
• **KUBO, Anna-Liisa
10134 Tallinn (EE)**
• **VASILIEV, Grigory
13419 Tallinn (EE)**

(74) Representative: **Berggren Oy
P.O. Box 16
Eteläinen Rautatiekatu 10A
00101 Helsinki (FI)**

(56) References cited:
**EP-A1- 3 789 046          WO-A1-2012/162557
US-A1- 2016 022 606**

• **BALLOTTIN DANIELA ET AL: "Elucidating Protein Involvement in the Stabilization of the Biogenic Silver Nanoparticles", NANOSCALE RESEARCH LETTERS, vol. 11, no. 1, 1 December 2016 (2016-12-01), US, XP055944214, ISSN: 1931-7573, Retrieved from the Internet <URL:https://www.ncbi.nlm.nih.gov/pmc/articles/PMC4927534/pdf/11671_2016_Article_1538.pdf> DOI: 10.1186/s11671-016-1538-y**

(52) Cooperative Patent Classification (CPC): (Cont.)

C-Sets
**A01N 25/34, A01N 59/16, A01N 59/20;**
**A01N 59/16, A01N 59/20**

**Description**

This invention is related to antimicrobial compounds and materials, especially antibacterial and antiviral compounds and materials, and antibacterial dressings, and to uses of the same.

Development of new antibacterial and antiviral compounds is one of the top priorities flagged by the World Health Organization, WHO. In SARS-CoV-2 era, there is a growing demand for advanced antiviral medical devices, textiles and personal protective equipment. The most critical target group of novel antibacterial compounds are multidrug-resistant bacterial strains that cause infections in the patients with injuries (such as burns, traumatic wounds, surgery etc.) or underlying medical conditions exemplified by pressure ulcers and diabetes. Every fifth diabetic develops chronic wounds, commonly foot ulcers that are frequently infected by bacteria. In 15-27 % cases bacterial wound infection leads to the gangrene that requires an amputation of the leg. This demonstrates that current wound infection management strategies are not efficient enough, and there is a great need for new remedies.

Current wound infection management strategies mainly rely on systemic antibiotics and topical wound care, including antibacterial creams and wound dressings. Systemic antibiotic treatment is a temporary measure and is further complicated by the fact that about 30% of the most common wound bacterial isolates (*Staphylococcus aureus, Pseudomonas aeruginosa, Escherichia coli*) are multidrug-resistant. Instead, or in addition to antibiotics, antibacterial wound dressings are applied locally (topically).

Metallic Ag has been used as antimicrobial since ancient times and it possesses broad-spectrum antibacterial action, being currently the "gold standard" antibacterial metal. The vast majority of commercial antibacterial medical devices consist of silver (Ag), mostly in ionic or metallic form.

Utilization of Ag nanoparticles instead of ionic/metallic Ag enables controlling Ag ion release, to prevent rapid inactivation of Ag ions by the wound environment, to decrease the probability of bacterial resistance and to enhance the antibacterial effect of Ag ions.

Current wound dressings mainly rely on ionic/metallic Ag. Wound dressing Acticoat® with nanocrystalline Ag is one of the few examples that rely on Ag NPs.

Various antibacterial dressings comprising ionic Ag have been disclosed in patent publications: Cellulose fibers with ionic Ag is disclosed in US6471982B1; WO2003053484 discloses a textile matrix comprising metallic silver coated fibers, polyurethane and alginate with ionic Ag is disclosed in US20030021832A; cellulose and alginate with ionic Ag is disclosed in US8058499B2; nylon plated with metallic Ag is disclosed in US20030176827A1.

There are also publications available disclosing use of Ag nanoparticles, e.g. EP1901723A2 discloses polyethylene fibers with Ag nanoparticles. US 9838652 similarly discloses an Ag nanoparticle composition for preventing tissue infections.

Even if for example EP1901723A2 discloses Ag NP containing dressings to provide faster healing time and bacterial clearance compared to antibiotic sulfadiazine and lower frequency of wound sepsis and secondary bacteremias in burn wounds compared to Ag salt ($AgNO_3$) in randomized controlled clinical trials, they still do not inactivate bacteria and especially bacterial biofilms entirely.

In addition, according to various experiments and publications, Ag has low effect against coronavirus as is also recently demonstrated by our research group. We tested a range of Ag nanoparticles and Ag salt against influenza virus and coronavirus. Surprisingly, the half maximal effective concentrations ($EC_{50}$) of these tested silver compounds to influenza as well coronavirus were >50 mg/l indicating very low antiviral effect.

Copper as such is known to have weak antimicrobial effects as compared to silver. For this reason, using copper in addition to or instead of Ag, which is known for long to have good antimicrobial efficacy, is not widely used in antibacterial medical devices such as wound dressings.

However, there are some publications providing combinations of Ag and Cu in combination with other metals. For example, US20160022606 discloses a wound care system, where the system includes a mixture of colloidal silver, copper and zinc oxides.

WO2012162557 is directed to a composition having antimicrobial activity comprising particles comprising at least one inorganic copper salt, CuI and at least one functionalizing agent in contact with the particles, the functionalizing agent stabilizing the particles in a carrier such that an antimicrobially effective amount of ions are released into the environment of a microbe. The composition may further comprise at least one of silver particle or a silver halide particle which may be functionalized with an amino acid.

There are growing concerns about ecotoxicity of Ag and Ag NPs and their adverse effects in *vitro* and *in vivo* in patients, including allergies. Therefore, there is a great need for safer (e.g., with less silver content) and more efficient antibacterial dressings, fabrics and other materials that would inactivate bacteria and their biofilms efficiently, act against various bacterial species, including antibiotic-resistant strains and inactivate viruses, including influenza virus and coronavirus; dressings that would provide faster wound re-epithelization, especially for diabetic ulcers, efficiently healing or preventing bacterial infection in chronic wounds (such as diabetic wounds, pressure ulcers) and in acute wounds (such as burns, traumatic and surgical wounds) and therefore preventing the limb amputations, mortality and days spent in hospital.

Accordingly, this disclosure solves the above-mentioned problems. The scope of invention is defined by the claims.

It is an object of this disclosure to provide antimicrobial materials, compositions and related solutions for medical devices, including personal protective equipment. Especially an object is to provide antibacterial and antiviral materials.

It is an object of this invention to provide an antimicrobial medical device (such as wound dressing, patch, face mask, ointment, cream, spray, catheter) comprising a silver component and a copper component, wherein the combination of silver and copper components provide a synergistic antimicrobial effect, thus leading to enhanced wound healing efficacy, faster inactivation of bacteria, reduced inflammation and hence, better outcome of wound infection such as reduced probability of limb amputation due to bacterial infection. In addition, due to the synergy of silver and copper components according to this disclosure, concentration of silver may be significantly decreased (reducing adverse effects associated with frequent use of silver-based products) without losing antimicrobial efficacy.

The silver component comprises Ag nanoparticles but may also comprise ionic silver in addition to the nanoparticles.

The copper component comprises copper- based salts selected from $CuSO_4$.

According to certain embodiments the copper and silver components are provided in or on a medical device in such amounts that based on $mg/cm^2$ the Cu/Ag ratio in the antimicrobial device (e.g., dressing or facemask) is between 1 to 70, more preferably between 1 to 20 and most preferably between 1 to 8. According to certain embodiments a dressing may have Ag -concentration of $0.1$-$10$ $mg/100cm^2$, preferably, $0.5$-$3$ $mg/100$ $cm^2$ and most preferably $0.6$-$1.0$ $mg/100$ $cm^2$. The Cu-concentration may be $0.5$-$7.5$ $mg/100$ $cm^2$ and more preferably $2.5$-$3.0$ $mg/100$ $cm^2$. The Ag-concentration is preferably 1-2% of the contact layer, and the Cu concentration is preferably 3-5% of the contact layer.

According to certain embodiments the silver and copper compounds are provided in or on the medical device such that they create a synergistic antimicrobial effect which can be determined by coefficient of antimicrobial synergy K(Syn) calculated as:

$$K(Syn) = 1/ \left( \frac{\text{MBC of Ag compound in mix}}{\text{MBC of Ag compound alone}} + \frac{\text{MBC of Cu compound in mix}}{\text{MBC of Cu compound alone}} \right),$$

wherein the K(syn) is higher than 1, preferably higher than 2, more preferably higher than 3, and most preferably higher than 4. Notably, K(Syn) as defined here may as well be used to measure antiviral synergy.

It is an object of this invention to provide an antimicrobial medical device comprising Ag and $CuSO_4$ and their combinations in a ratio such that Cu/Ag ratio is between 1 to 70, more preferably between 1 to 20 and most preferably 1 to 8.

According to certain embodiments the medical device comprises Ag nanoparticles either non-functionalized or functionalized with one or more groups selected from carboxyl functional groups (-COOH), quaternary ammonium, polyethylene imine, branched polyethylene imine, PEG, citrate, PVP, dextran coating, amine groups ($-NH_2$), amino acids, bacteria binding peptides, polyoxometalates (POMs), antibodies, their fragments, and combinations thereof.

According to certain embodiments the medical device, for example an antimicrobial dressing or facemask comprises protein stabilized Ag nanoparticles and optionally ionic silver, and copper compounds comprising the above Cu based salts.

According to certain embodiments the antimicrobial dressing comprises a skin contact layer and an absorbant layer, the skin contact layer comprising the Ag nanoparticles and the copper compounds, and the absorbant layer is a natural or synthetic polymer.

It is an object of this invention to provide an antimicrobial dressing comprising Ag nanoparticles and Cu-compounds in a ratio such that Cu/Ag ratio is between 1 to 70, more preferably between 1 to 20, most preferably between 1 to 8, and wherein coefficient of antimicrobial synergy when measured as:

$$K(Syn) = 1/ \left( \frac{\text{MBC of Ag NPs in mix}}{\text{MBC of Ag NPs alone}} + \frac{\text{MBC of copper compound in mix}}{\text{MBC of copper compound alone}} \right)$$

MBC of Ag NPs alone MBC of copper compound alone is higher than 1, preferably higher than 2, more preferably higher than 3, and most preferably higher than 4.

It is an object of this invention to provide antimicrobial materials that are suitable for face masks and/or other protective wearable equipment to prevent exposure to airborne microbes, especially to viruses. It is an object to provide materials especially suitable for face masks and capable of killing coronaviruses (such as SARS Cov-2) and influenza (such as H1N1) viruses.

According to certain embodiments a filter material of a medical mask comprises Ag-nanoparticles and $CuSO_4$ and the mask is capable of reducing activity or inactivating SARS-CoV-2 and/or H1N1 virus.

It is a further object of the invention to provide an antimicrobial formulation comprising Ag-nanoparticles and $CuSO_4$ in such a ratio that Cu/Ag ratio is between 1 to 70, more preferably between 1 to 20 and most preferably 1 to 8

According to certain embodiments the antimicrobial formulation comprises Ag-nanoparticles selected from either non-functionalized or functionalized with one or more groups selected from carboxyl functional groups (-COOH), quaternary ammonium, polyethylene imine, branched polyethylene imine, PEG, citrate, PVP, dextran coating, amine groups ($-NH_2$), amino acids, bacteria binding peptides, polyoxometalates (POMs), antibodies, their fragments, and combinations thereof.

According to certain embodiments the antimicrobial formulation comprising Ag nanoparticles and $CuSO_4$ in such a ratio that Cu/Ag ratio is between 1 to 70, more preferably between 1 to 20 and most preferably 1 to 8, and coefficient of antimicrobial synergy of the formulation when measured as

$$K(Syn) = 1 / \left( \frac{\text{MBC of Ag NPs in mix}}{\text{MBC of Ag NPs alone}} + \frac{\text{MBC of copper compound in mix}}{\text{MBC of copper compound alone}} \right)$$

MBC of Ag NPs alone MBC of copper compound alone is higher than 1, preferably higher than 2, more preferably higher than 3, and most preferably higher than 4.

According to certain embodiments the antimicrobial formulation is effective against bacteria, including multi-drug resistant isolates, fungi, protozoa, viruses, including SARS CoV-2 and H1N1, and pathological proteins, including $\alpha$-synuclein, prions, and amyloid beta.

According to certain embodiments it is provided the antimicrobial formulation for use in preventing or diminishing microbial growth on human tissue or wound dressing for use in preventing or diminishing microbial growth on human tissue, the formulation or wound dressing comprising a silver component and a copper component, wherein the silver component comprises Ag nanoparticles alone or in combination with ionic silver and the copper component comprises Cu-based salts selected from CuSO4 in such proportions that Cu/Ag ratio is between 1 to 70, more preferably between 1 to 20 and most preferably 1 to 8, and a coefficient of antimicrobial synergy is higher than 1, higher than 2, higher than 3 or higher than 4.

According to certain embodiments it is provided the antimicrobial material for use in inactivation of virus, preferably SARS-CoV-2 virus or H1N1 virus upon contact with the material, the antimicrobial material comprising silver and copper components, wherein the silver component comprises Ag nanoparticles alone or in combination with ionic silver and the copper component comprises Cu-based salts selected from CuSO4 in such proportion that Cu/Ag ratio is between 1 to 70, more preferably between 1 to 20 and most preferably 1 to 8.

According to certain embodiments the antimicrobial material comprising silver nanoparticles and $CuSO_4$ in such proportion that Cu/Ag between 1 to 70, more preferably between 1 to 20 and most preferably 1 to 8 is efficient as a face mask to prevent or slow spreading of microbes, especially SARS-CoV-2 virus and H1N1-virus due to inactivation of microbes upon contact with the material.

It is an object of this invention to provide the antimicrobial dressing for use in a method to cure or improve healing of a wound, the method comprising the step of administering the antimicrobial dressing on a wound, such that Cu/Ag ratio is between 1 to 70, more preferably between 1 to 20 and most preferably 1 to 8.

According to certain embodiments the antimicrobial dressing for use comprises silver compound and $CuSO_4$ in a ratio such that Cu/Ag ratio is between 1 to 70, more preferably between 1 to 20 and most preferably 1 to 8 and wherein coefficient of antimicrobial synergy when measured as

$$K(Syn) = 1 / \left( \frac{\text{MBC of Ag compound in mix}}{\text{MBC of Ag compound alone}} + \frac{\text{MBC of Cu compound in mix}}{\text{MBC of Cu compound alone}} \right)$$

MBC of Ag compound alone MBC of Cu compound alone is higher than 1, preferably higher than 2, more preferably higher than 3, and most preferably higher than 4.

It is an object of this invention to provide the antimicrobial formulation for use in a method to treat a wound, the method comprising the step of administering the antimicrobial formulation on a wound, the antimicrobial formulation comprising Ag compound and $CuSO_4$ in such a ratio that Cu/Ag ratio is between 1 to 70, more preferably between 1 to 20 and most preferably 1 to 8 and coefficient of antimicrobial synergy of the formulation when measured as

$$K(\mathbf{Syn}) = 1 / \left( \underline{\frac{\text{MBC of Ag compound in mix}}{\text{MBC of Ag compound alone}}} + \underline{\frac{\text{MBC of Cu compound in mix}}{\text{MBC of Cu compound alone}}} \right)$$

MBC of Ag compound alone MBC of Cu compound alone is higher than 1, preferably higher than 2, more preferably higher than 3, and most preferably higher than 4.

According to certain embodiments additional metal-based nanoparticles or metal ions selected from Al, Au, Ce, Co, Fe, Ga, Ir, Mo, Ti, Zn may be added to the dressing or the formulation.

The advantages provided by the invention disclosed and claimed here includes among others a synergy of two antimicrobial components such that bacterial film is completely removed with several times lower silver concentrations than any known solution. The advantages include faster wound healing compared to dressings currently in use and the reduction of silver amount in wound treatment.

The advantages provided by this invention also includes a high efficacy against a large variety of microbes, including antibiotic resistant bacterial strains and their biofilms as well as viruses, especially SARS-CoV-2 and H1N1.

SHORT DESCRIPTION OF THE FIGURES

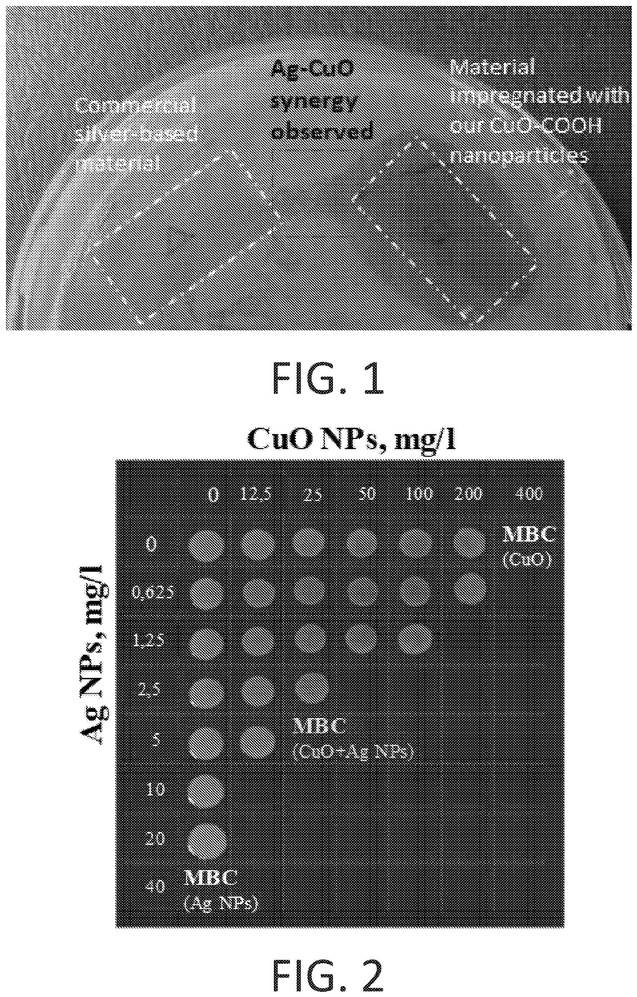

Fig 1 illustrates the synergy of Ag NPs and CuO NPs against *Escherichia coli* K12. Test was performed using slightly modified ISO 20645 (Textile fabricsDetermination of antimicrobial activity -- Agar diffusion plate test). Commercial silver-based wound care product (Hansaplast) is on the left, in-house prepared wound care material containing CuO-COOH NPs is on the right. Material contact area with bacteria (surface area = 2.5 cm$^2$) is denoted by the white dashed line; unexpected bacteria-free area suggesting the synergy between commercial silver material and our nano copper-based material is denoted by the red dashed line.

Fig. 2 is an example of synergy of NPs. E. *coli* suspension was incubated with different concentrations of either Ag NPs, CuO NPs or their combinations for 24 h, and 3 $\mu$L of bacteria- NP mixture was pipetted onto agarized broth.

Fig. 3 A and B show the synergistic effect of CuO NPs and CuSO$_4$ with Ag NPs (A) or AgNO$_3$ (B) to a range of Gram-negative and Gram-positive bacteria commonly colonizing wounds. Bacterial suspension was incubated with Ag NPs, CuO NPs, respective ions or their combinations for 24 h, plated on agarized broth, and MBC was determined.

Fig 4. Shows the synergistic effect of CuO NPs and CuSO4 with various Ag NPs E. *coli* K12 suspension was incubated with Ag NPs, CuO NPs, respective ions or their combinations for 24 h, plated on agarized broth, and MBC was determined.

Fig. 5 shows coefficient of antibacterial synergy between Ag NPs and copper components depending to Cu/Ag ratio (calculated on the basis of metal) in the mixture.

Fig. 6 A and B Scratch closure (re-epithelization) of BALB/c fibroblasts in vitro after 24-h exposure to the control (medium alone) or medium with copper.

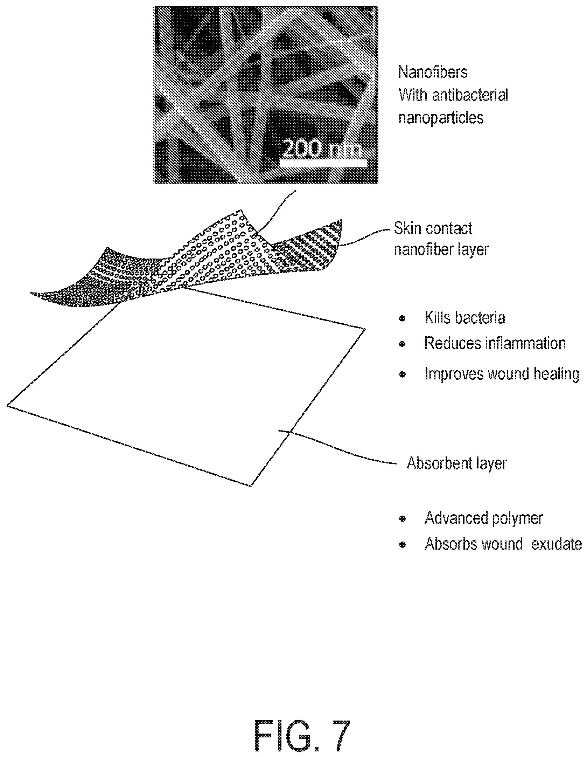

Fig 7. Illustrates the design of the wound dressing containing an antibacterial skin contact layer and an absorbant layer according to this disclosure. Nylon 6 was dissolved in formic acid at various concentrations (15-20 wt %) and the solutions were stirred overnight at magnetic heated plate stirrer at 40°C to ensure the full dissolution. 0-2000 ppm Cu-AgNP suspensions were stirred with nylon 6-formic acid for 1 h at 40° and then subjected to the electrospinning as described by Javed et al 2. The polymer solutions were electrospun with a fixed mass flow rate of 0.12 mL/h and a voltage of 15 kV for 2 hours. After preparation of the fibers, scanning electron microscopy was used to determine the morphology and the average diameter of the nanofibers (Fig insert).

Fig 8. Shows scanning transmission electron microscope images coupled with high-angle annular dark-field (HAADF) and Energy Dispersive X-Ray Analysis (EDX) demonstrated presence of Cu and Ag NPs in the electrospun fibers.

Fig 9. Shows antibacterial efficiency of electrospun skin contact layer with CuSO4, Ag NPs or their combinations against *E.coli* K12. Concentrations are recalculated on metal (Ag or Cu) content to enable comparison.

Fig 10. Shows improved antibacterial efficiency (larger zone of inhibition) of the wound dressing according to this disclosure with CuSO$_4$ and Ag NPs against antibiotic-resistant *Pseudomonas aeruginosa* compared to the popular commercial antibacterial wound dressings. Legend: 1: Our wound dressing: skin layer of silk fibroin with CuSO$_4$ and Ag NPs and absorbant layer; 2: commercial wound dressing 1 and 3: commercial wound dressing 2. The darker color indicates growth of bacteria.

Fig. 11 A-B demonstrates representative Petri dishes with bacteria isolated from wounds of rats after 24-h treatment with placebo (dressing without antibacterial components) (1), Aguacel Ag+ (2), or a wound dressing according to this disclosure (AAWD= advanced antibacterial wound dressing) prepared on the basis of Cu-Ag nanoparticle combination ; n=27.

Fig. 12 shows the number of *P.aerunginosa* cells in wound swab on different days in experiments conducted with rats.

Fig. 13 Wound healing progress after 5-day treatment with different wound dressings in rat wound infection model. Representative images from blind histology of tissue biopsy after treatment with placebo (A), Aquacel Ag+ (B) or the wound dressing according to this disclosure (C). n=27. There was lower number of inflammatory cells (leukocytes) and more mature epidermis and collagen tissue after the treatment with wound dressing disclosed here..

Fig. 14. A-D shows antiviral activity of material according to this disclosure with Ag and Cu nanoparticles against SARS-CoV-2 virus (A and B) and against H1N1 virus (C and D).

DETAILED DESCRIPTION OF THE INVENTION

*Definitions:*

*Nanoparticle* as used in this disclosure means a particle of matter that has at least one dimension between 1 and 1000 nm in diameter, preferably between 1 and 100 nm, more preferably between 1 and 60 nm, and most preferably between 1 and 40 nm. In case the particle is in the form of a fiber or a tube, the diameter of the fiber or tube is between 1 and 100 nm, preferably between 1 and 60 nm and most preferably between 1 and 40nm regardless of the length of the tube or fiber.

*Antibacterial Synergy* as used in this disclosure means interaction or cooperation of two or more compounds that produce a combined antibacterial effect greater than the sum of their separate effects. The antibacterial synergy is measured as Coefficient of antibacterial synergy K(Syn). According to this disclosure there is antibacterial synergy when the coefficient is higher than 1, more preferably higher than 2, even more preferably higher than 3, and most preferably higher than 4.

*Surface functionalization of nanomaterials* as used in this disclosure refers to assembling different organic and/or inorganic materials together in nanoscale through covalent bonds or noncovalent bonds including hydrogen bonds, electrostatic force, or van der Waals force.

*A formulation is effective against microbes* as used in this disclosure means that treatment with the formulation is capable of killing at least 90%, preferably at least 99%, more preferably 99.9%, and most preferably more than 99.99% of the microbes.

*Silver compound,* as used in this disclosure includes Ag nanoparticles alone, or in a combination with ionic or metallic silver.

*Copper compound,* as used in this disclosure includes Cu nanoparticles, Cu salts or combinations thereof.

*Medical device,* as used in this disclosure include personal protective devices and generally means devices, structures, and compositions intended for use in the diagnosis of disease or other conditions, or in the cure, mitigation, treatment, or prevention of disease in a living organisms, preferably in a mammal, most preferably in a human being. The term medical device thus includes for example stents, catheters, abdominal plugs, adhesive films, contact lenses, bandages, face masks, textiles, filter materials, injectors of drugs, wound dressings, patches, ointments, creams, sprays, without limiting the definition to these examples.

*MBC,* minimum bactericidal concentration is the lowest concentration of Ag or Cu compounds required to kill particular bacterium. In reference to antiviral effect MBC would mean the minimum concentration of Ag or Cu compounds require to kill particular virus.

This disclosure provides combinations of Ag nanoparticles (Ag NPs) alone or in combination with ionic silver, and Cu compounds comprising Cu salts, selected from CuSO4, in medical devices, dental materials, antimicrobial dressings, antibacterial fabrics. Even if Cu is known to have antimicrobial effects, its use as antibacterial compound in medical devices or wound dressings has been limited due to the fact that its antimicrobial effects are known to be considerably weaker than those of silver. It was now surprisingly and unexpectedly found that combining Ag NPs with the $CuSO_4$ the antimicrobial effects are 3 to 5 times higher than with Ag NPs alone. This allows making medical devices, dental materials, antibacterial dressings, and other applications while using less Ag. One preferred embodiment is a wound dressing containing less Ag than wound dressings that are commercially available with higher antimicrobial efficacy and improved wound healing properties. In referring to the appended figures various embodiments are now described.

In the following examples and tests, only combinations of Ag NPs are part of the invention.

Referring now to Figure 1 a comparison between antimicrobial efficacy of a dressing with silver nanoparticles (Ag NPs) and copper oxide nanoparticles (CuO NP) was conducted as an agar diffusion plate test. A commercial wound care material incorporating silver nanoparticles and in-house produced wound care material incorporating copper oxide nanoparticles (CuO NPs) were placed on a same agar plate. The results shown in Figure 1 establish the initial surprising and unexpected observation that in the area between the two materials there was a bacteria-free area.

In order to investigate this synergy effect further, detailed experiments were conducted.

A synergistic effect between CuO NPs and Ag NPs was demonstrated in an experiment shown in Figure 2. Antimicrobial efficacy of NPs was compared by determining minimal bactericidal concentration (MBC, the lowest tested concentration of NPs yielding no visible bacterial growth). As can be seen from figure 2 already 10 mg/l Ag NPs + 12.5 mg/l

7

CuO NPs efficiently inactivated E. *coli,* when used in combination, while the MBC was as high as 40 mg/l for Ag NPs and 400 mg/l for CuO NPs. This clearly proves the synergistic effect.

In order to quantify and compare the synergistic effect between different Ag and Cu compounds and among various bacterial strains, we introduced the term "Coefficient of antimicrobial synergy", K(Syn), showing the antimicrobial efficiency of Ag compound and Cu compound combinations (mix) compared to the sum of the MBC values of individual compounds and calculated as follows:

$$K(\mathbf{Syn}) = 1/ \left( \frac{\text{MBC of Ag compound in mix}}{\text{MBC of Ag compound alone}} + \frac{\text{MBC of Cu compound in mix}}{\text{MBC of Cu compound alone}} \right)$$

It is to be understood that in case silver compound includes NPs only and the Cu compound includes CuO NPs only the equation can be written:

$$K(\mathbf{Syn}) = 1/ \left( \frac{\text{MBC of Ag NPs in mix}}{\text{MBC of Ag NPs alone}} + \frac{\text{MBC of CuO NPs in mix}}{\text{MBC of CuO NPs alone}} \right)$$

This disclosure provides a wound dressing comprising silver compounds comprising Ag nanoparticles alone or in combination with ionic silver and copper compounds selected from copper-based salts, selected from $CuSO_4$.

The dressing preferably comprises the Ag-nanoparticles and the copper-based salts, and their combinations in a ratio such that Cu/Ag ratio is between 1 to 70, more preferably between 1 to 20 and most preferably 1 to 8. The combination of Ag nanoparticles and Cu compounds as described here provides an unexpected synergy between the Ag nanoparticles and Cu compounds resulting antimicrobial effects 3 to 5 times higher than Ag nanoparticles alone. Cu alone is a weak antimicrobial compound and was not expected to increase the effect of Ag nanoparticles.

The antimicrobial synergy is measured by a coefficient of antibacterial synergy defined as K(Syn) (see above).

According to certain embodiments the synergistic effect is used to make highly efficient antibacterial wound dressings.

According to certain embodiments the synergistic effect is used to various wound care treatment products such as woven or non-woven wound dressings, cotton gauzes, hydrofibers, gels, creams, ointments, hydrogels, hydrocolloids, foams, films, patches, pads to enhance their antimicrobial efficiency and anti-inflammatory and wound healing properties. For example, NP combination can be incorporated into or combined with different synthetic or natural polymers such as alginate, carboxymethyl cellulose, cellulose, cellulose ethyl sulphonate chitosan, chitin, fibroin, nylon, polyurethane, silicone, paraffin, polyethylene or polyvinyl with or without additional components to produce fibers, woven or non-woven wound dressings, cotton gauzes, hydrofibers, gels, creams, ointments, hydrogels or foams produced by sonochemistry, plating, electrospinning, freeze-drying, deposition or other techniques. Additional components may include Al, Au, Ce, Co, Fe, Ga, Ir, Mo, Ti, Zn as nanoparticles or as salts.

According to further embodiments the synergistic effect can be used in topical treatment of chronic or acute skin damage caused by infection, injury, inflammation, or medical condition: acne, herpes, skin irritations, inflammations excoriations or abrasions, traumatic injuries, leg and pressures ulcers, burns and surgical, necrotic, chronic or neuropathic/diabetic wounds, graft sides, under compression bandages.

According to even further embodiments the synergistic effect can be used in antimicrobial agents in medical devices like stents, catheters, abdominal plugs, adhesive films, face masks, contact lenses, lens cases, bandages, injectors of drugs and other devices that pass through the skin or contact with the skin, as well as prostheses, spokes, screws, and other implants introduced into the body.

According to further embodiments the synergistic effect can be used in antimicrobial, preservative, antioxidative or regenerating components in cosmetics: creams, ointments, lotion, sprays, lipsticks, gels, soaps, shampoos, scrubs.

According to even further embodiments the synergistic effect can be used in antimicrobial component for disinfecting in liquids: for washing and disinfecting hands, wounds, surfaces, and items.

According to still further embodiments the synergistic effect can be used in textiles to reduce the number of microorganisms and reduce the unpleasant smell from the fabric.

According to even further embodiment the synergistic effect can be used in antimicrobial components used in dentistry for disinfection on antimicrobial component for dental materials.

According to certain embodiments the synergistic effect can be used in anti-inflammatory components or additives for the treatment of local and systemic autoimmune and allergic diseases, including psoriasis, allergic and non-allergic dermatitis, acne, pemphigus, and pemphigoid.

According to certain embodiments the synergistic effect can be used in antimicrobial components of the material printed on 3D printers or produced by other technologies and afterwards implanted into the body; electrospun tissue scaffolds part of the nanorobots and electronic devices injected into blood or into organs or used on the body surface.

According to certain embodiments the medical device of this disclosure may be a dressing or a formulation comprising a first and a second component. The first component is an Ag-based component, and the second component is a Cu-based component. The first component comprises an Ag based nanoparticle, or the first component may be a combination of Ag salt and Ag based nanoparticle. The second compound comprises a Cu salt, selected from $CuSO_4$.

According to certain embodiments further components may be added to the medical device, dressing and/or formulation. For example, a third component may include Al, Au, Ce, Co, Fe, Ga, Ir, Mo, Ti, Zn as nanoparticles or as salts.

The Ag based nanoparticles may be functionalized or non-functionalized. They may be stabilized by surfactants, proteins, polyvinyl pyrrolidone (PVP), citrate, polyethylene glycol (PEG), however this is not a limiting list, but other stabilizers may also be used. The Ag NPs may be modified with one or several surface functionalizations, non-limiting list of functional groups includes carboxyl functional groups (-COOH), quaternary ammonium, polyethylene imine, branched polyethylene imine, PEG, citrate, PVP, dextran coating, amine groups (-$NH_2$), amino acids, bacteria binding peptides, polyoxometalates (POMs), antibodies, their fragments, and combinations thereof.

The copper-based component comprises a copper salt, selected from $CuSO_4$. The copper-based component may further comprise copper (oxide)-based nanoparticle. The optional nanoparticles may be CuO- or $Cu_2O$- nanoparticles but other nanoparticles may also be used. The surfaces of the nanoparticles may be functionalized or not. A non-limiting list of functionalizing groups includes carboxyl groups, PEG, citrate, PVP, dextran coating, amine groups (-$NH_2$), proteins, quaternary ammonium, polyethylene imine, branched polyethylene imine, peptides, POMs, antibodies, their fragments, and combinations thereof. The copper compound $CuSO_4$ has a positive charge.

In order to quantify and compare the synergistic effect between different Ag NPs and Copper compounds among various bacterial strains, we introduced the term "Coefficient of antibacterial synergy", K(Syn), showing the antimicrobial efficiency of Ag- and copper compound combinations (mix) compared to the sum of the MBC values of individual Ag- and Cu compounds and calculated as follows:

$$\mathbf{K(Syn)} = 1/ \left( \frac{\text{MBC of Ag compounds in mix}}{\text{MBC of Ag compounds alone}} + \frac{\text{MBC of Cu compound in mix}}{\text{MBC of Cu compound alone}} \right)$$

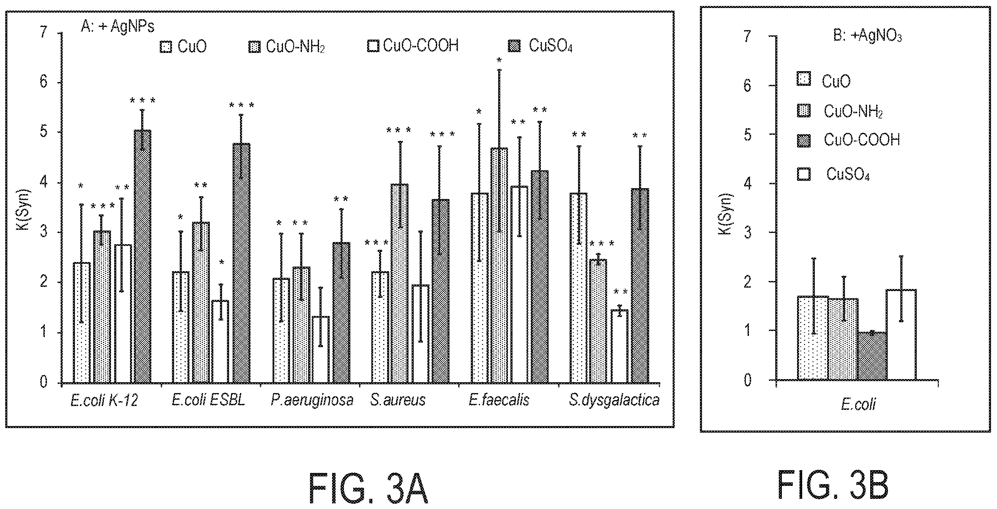

Bacterial suspensions were incubated with Ag NPs, CuO NPs, respective ions or their combinations for 24 h, plated on agarized broth, and MBC was determined. The bacterial strains included *Streptococcus aureus, Pseudomonas aeruginosa, Escherichia faecalis, Staphylococcus dysgalactiae* and *Escherichia coli*. Results are shown in Figure 3. Synergy between Ag NPs and CuO NPs with different surface functionalizations was observed in case of all tested bacterial strains and was the highest for CuO NPs with the positive surface charges (CuO and especially CuO-$NH_2$ NPs) and for positively charged $CuSO_4$ (ionic metal) (*Fig 3A*), suggesting that positive charges enhanced synergy. In line with this hypothesis, the lowest K(Syn) was observed for negatively charged CuO-COOH NPs. When ionic silver (as $AgNO_3$) was used instead of Ag NPs, synergistic effect was considerably lower. Even if ionic silver salt alone with the copper compounds was not specifically efficient demonstrating that the surface of Ag NPs is pivotal for the enhanced synergistic effect (*Fig 3B*), the combination of Ag NPs with ionic or metallic silver with Cu compounds can still provide a substantially high antimicrobial synergy (not shown).

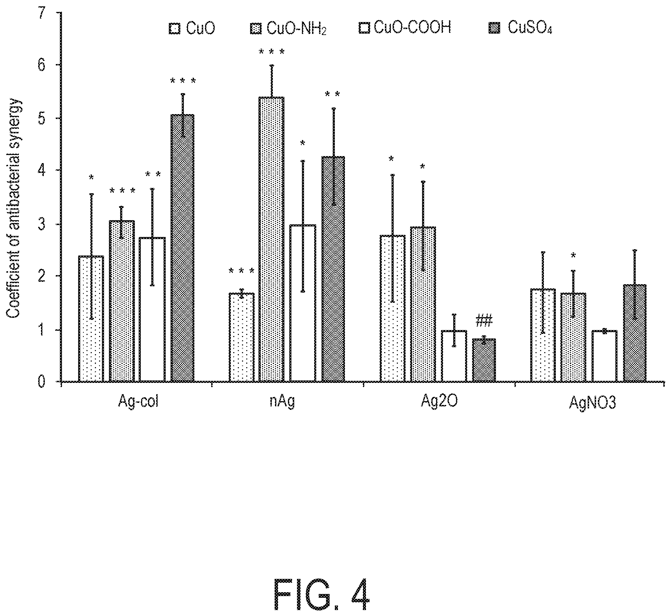

Three different Ag NPs (size, surface functionalizations) were tested for synergy with Cu compounds. In figure 4 AgNP1 is colloidal protein-functionalized silver, AgNP 2 is unfunctionalized metallic silver, and Ag NP3 $Ag_2O$. Test species here was E. *coli* K12. *(Fig 4)*. E. *coli* suspension was incubated with Ag NPs, CuO NPs, respective ions or their combinations for 24 h, plated on agarized broth and MBC was determined. Synergy with positively charged CuO NPs in case of all three tested Ag NP types was observed. This suggests synergistic effect is universal.

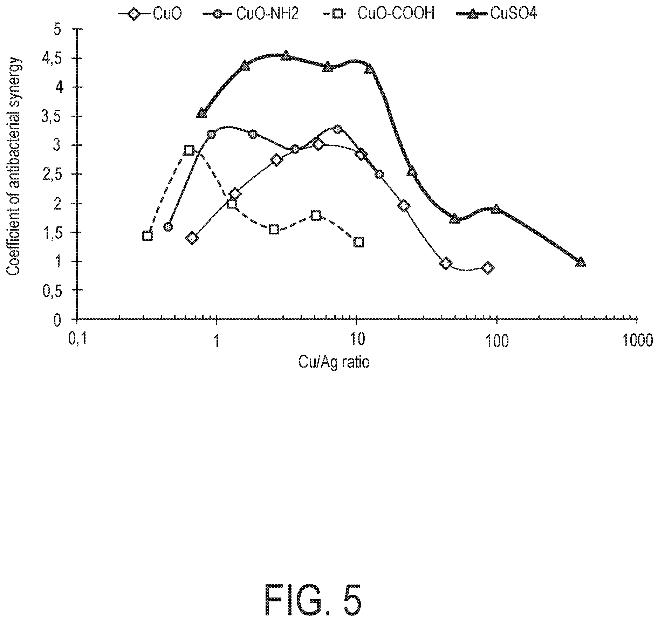

Experiments were conducted to define the optimal range of the metal components to achieve highest coefficient of antimicrobial synergy K(Syn). Figure 5 shows that a synergy is highest when Ag NPs are applied with CuSO4 and the Cu/Ag ratio is between 1 and 10. However, ratios being as high as 70-100 still provided substantial synergy benefit. Similarly, by using CuO $NH_2$ provided a good synergy with Ag NPs at Cu/Ag ratio being 1 to 10. With CuO and Ag NPs the synergy was best when Cu/Ag ratio was 7 to 10, and with CuO-COOH and Ag NP the best synergy was achieved when Cu/Ag ration was about 0.7. Accordingly, it seems that the combination of CuSO4 and Ag nanoparticles provides the best synergy and enable decreasing the amount of silver in the dressing.

Experiments were made to test the efficiency of selected NP combination (Ag NPs + CuO-$NH_2$ NPs, 1:4) on a range of multidrug-resistant clinical bacterial isolates. Experiments showed that NP combination was efficient against all tested bacterial isolates and pathogenic yeast *Candida albicans* according to slightly modified ISO20645:2004 standard: *E. coli* ATCC 25922 ; *E. coli* ATCC 13846; *E. faecalis* ATCC 29212; *E. faecalis* ATCC 51299 VRE(+); *K. pneumoniae* ATCC 700603 ESBL(+) ; *S. aureus* ATCC 25923 *S. aureus* NCTC 12493 MRSA(+) - *S. epidermidis ATCC* 12228;*P. aeruginosa* ATCC *27853; S.epidermidis* L20030902207; E. *coli* ESBL(+) L20030700513 ;*C. albicans* L20030700230

Test was performed according to modified ISO 20645:2004. Bacterial suspension (0.5 McFarland) was plated onto nutrient broth, and Whatman paper discs impregnated with 100 μl of nanoparticle combination (0.067% Ag NPs and

0.15% CuO-NH$_2$ NPs were placed on top. After 24-h the area under the discs was visually inspected.

According to ISO20645:2004, "good effect" is assigned to the antibacterial material that renders no growth under material. Good effect was verified with all the above strains.

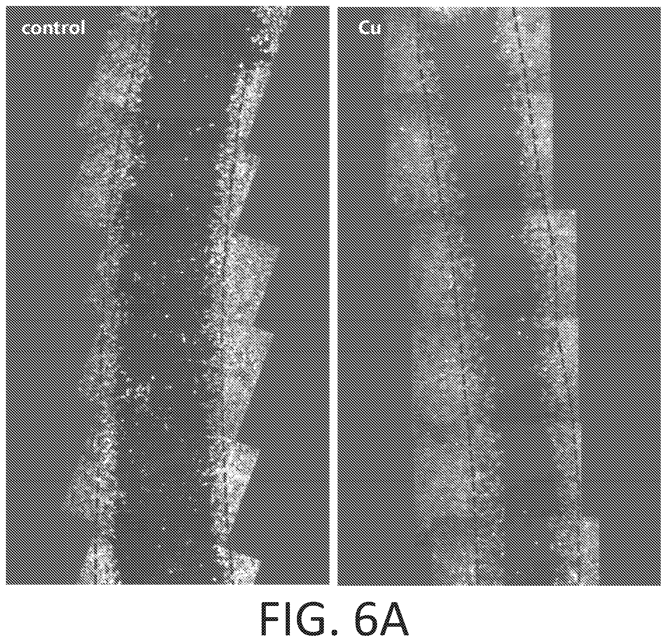

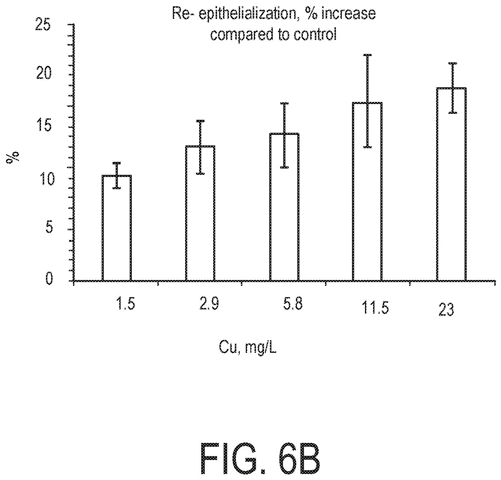

The potential of Cu component to support the wound healing using BALB/c fibroblasts and *in vitro* scratch assay as a proxy for the wound healing was tested. The test was conducted as described in Chun-Chi Liang, Ann Y Park & Jun-Lin Guan. (2007). In vitro scratch assay: a convenient and inexpensive method for analysis of cell migration in vitro. Nature Protocols, 2: 329-333. As is shown in Figure 6a, b, Cu enhanced the migration of fibroblasts, showing the potential for the improved wound healing.

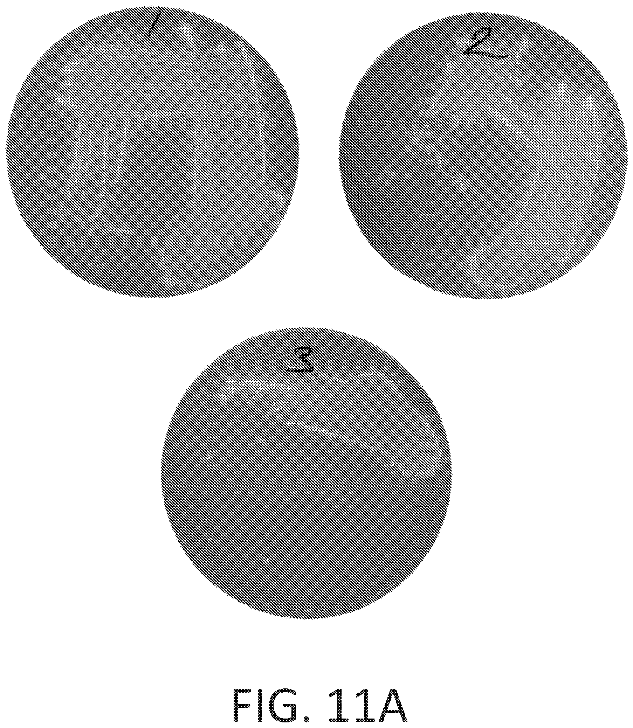

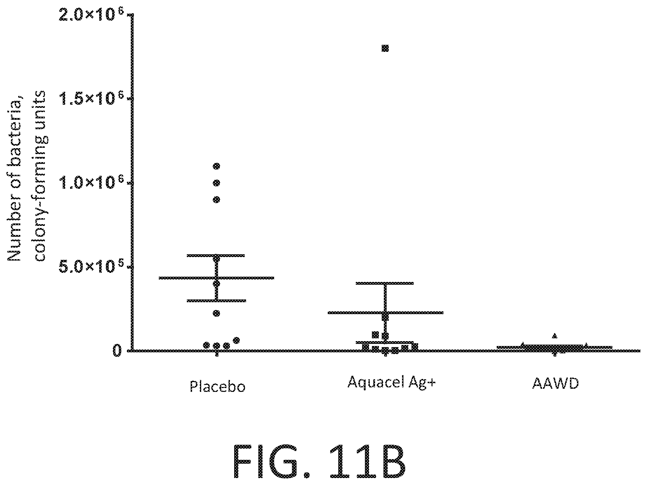

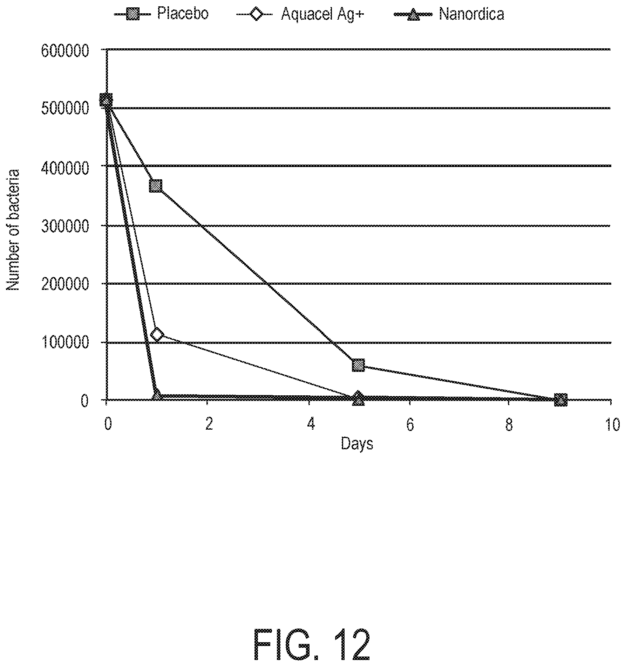

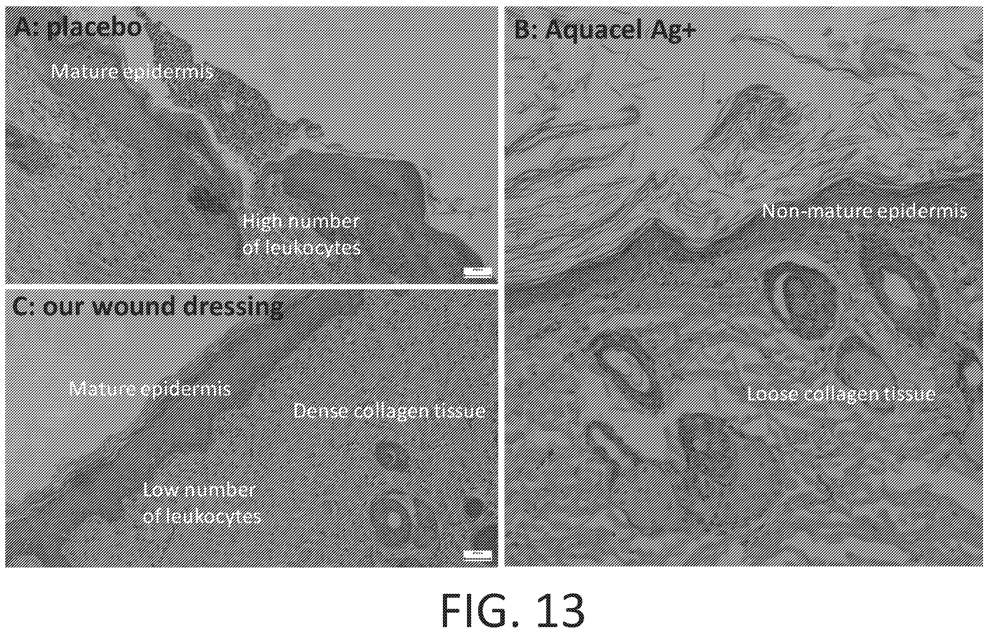

In further tests with animals, bacterial count in wound was found 26 times lower than using wound dressing without active components and 7.6 times lower than using Ag+ as active component only (cf. figure 11A and B). Moreover, number of pathogenic P. aeruginoa PAO1 was 40 times lower when the wound was treated with dressing comprising the AgNPs and Cu-compounds of this invention as compared to placebo (no active ingredients) and 12 lower than in wounds treated with dressings having only Ag+ as active ingredient (cf. Figure 12). It was found that the dressing comprising AgNPs and Cu-compounds as active ingredients supported formation of mature epidermis, and that new epithelium achieved after treatment of the dressing comprising AgNPs and Cu-compounds was more mature, more homogenous and less inflamed than epithelium achieved after treatment with only Ag+ containing dressing (Figure 13). Further advantages found in using the AgNP and Cu-compound comprising dressings were that neurophilis in blood of the test rats when measured on day 7 of the treatment was substantially lower than in rats treated with only Ag+ containing dressings (results not shown).

Accordingly, this disclosure provides an effective wound dressing that eliminates bacteria and supports the wound healing. The dressing comprises Ag compound including AgNPs alone or in combination with ionic Ag and Cu compounds. The Cu compounds comprise Cu salts. Cu salts are selected from CuSO$_4$. Optional Cu NPs are preferably CuO NPs. CuSO$_4$ and Ag NPs may be incorporated into fibers by electrospinning. Proposed technology implies that bacteria will be entrapped into the skin contact layer having a pore size of 100-300 nm, preferably approximately 200 nm, and become inactivated on the surface of the wound dressing, where moist wound bed will induce high local release of Cu ions and their interaction with AgNP surface.

Cu-Ag nanoparticles based wound dressings are safe and efficient when applied on patients (voluntary tests). They cause less side effects (like allergic reaction, dizziness, nausea, headache compared to competing commercial Ag-based dressing Aquacel Ag+). In addition, Cu-Ag compounds based wound dressings reduce bioburden, inflammation and support the wound healing, when applied on the wounds of patients with diabetic foot ulcer infection, patients with burns and beyond.

In in vitro tests the highest synergy was found when Ag NPs are applied with CuSO$_4$ and the Cu/Ag ratio is between 1 and 10. Similarly, by using CuO NH$_2$ provided a good synergy with Ag NPs at Cu/Ag ratio being 1 to 10. With CuO and Ag NPs the synergy was best when Cu/Ag ratio was 7 to 10, and with CuO-COOH and Ag NP the best synergy was achieved when Cu/Ag ration was about 0.7.

After performing efficacy and toxicity tests with rats, the best safety/efficacy balance was obtained when the dressing had Ag -concentration of 0.5-3mg/100cm$^2$ (1-2% of contact layer) and 3-7.5mg/100cm$^2$ of Cu (3-5% of contact layer). These concentrations were nontoxic for the mammalian fibroblasts and had an effective concentration of components against bacteria.

According to at least some embodiments the wound dressing comprises two layers (skin contact and absorbant layer). Ag compound and Cu compound are located in or on the skin contact layer. Cu compound may include Cu ion added as CuSO$_4$. (Fig. 7). Additional layers may be added. According to a preferred embodiment the absorbant layer is preferably alginate - a biocompatible and highly absorbant advanced polymer that is known in the art and used in treating chronic wounds. The alginate layer may comprise Cu-alginate gel.

The skin contact layer may be produced by electrospinning method enabling incorporation of NPs into polymers and getting tiny antimicrobial fibers According to certain embodiment Na-alginate is mixed with Ag NPs and Cu compounds in electrospinning solutions, and NPs-containing alginate fibers are produced by electrospinning. To enforce the strength of the fibers, additives such as polyethylene glycol or cellulose may be added. According to certain embodiments the skin contact layer has pore sizes between 100 and 300 nm, preferably 150-250 nm, and most preferably about 200 nm (i.e., 190-210 nm).

Accordingly, this disclosure provides antimicrobial medical devices for use in preventing or diminishing microbial growth on mammal, preferably human tissue. Especially the medical devices include antimicrobial dressings and formulations, but other variations are also possible. The antimicrobial medical devices are effective on a large number of microbes, including antibiotic resistant strains of various bacteria. The medical devices according to this disclosure are efficient against at least *Escherichia coli, Pseudomonas aeruginosa, Enterococcus faecalis, Staphylococcus aureus, Klebsiella pneumoniae, Staphylococcus epidermidis, Staphylococcus dysgalactiae and Candida albicans.*

Further experiments were conducted to verify the antiviral efficiency of the materials and compounds comprising Ag and Cu nanoparticles. Especially interest was to verify the antiviral efficiency against SARS-Cov2 and H1N1 virus.

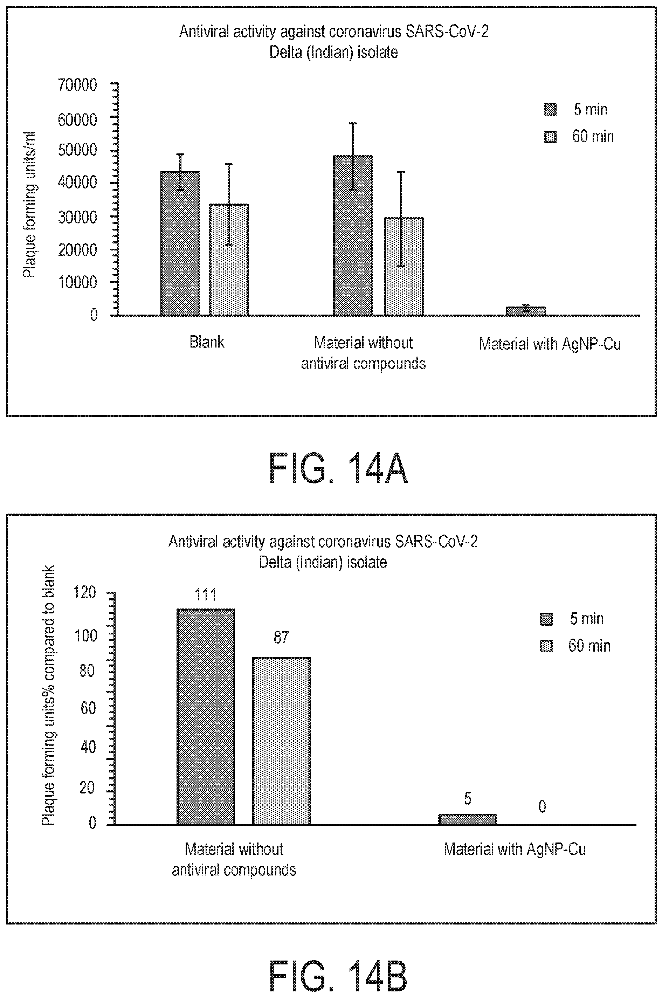

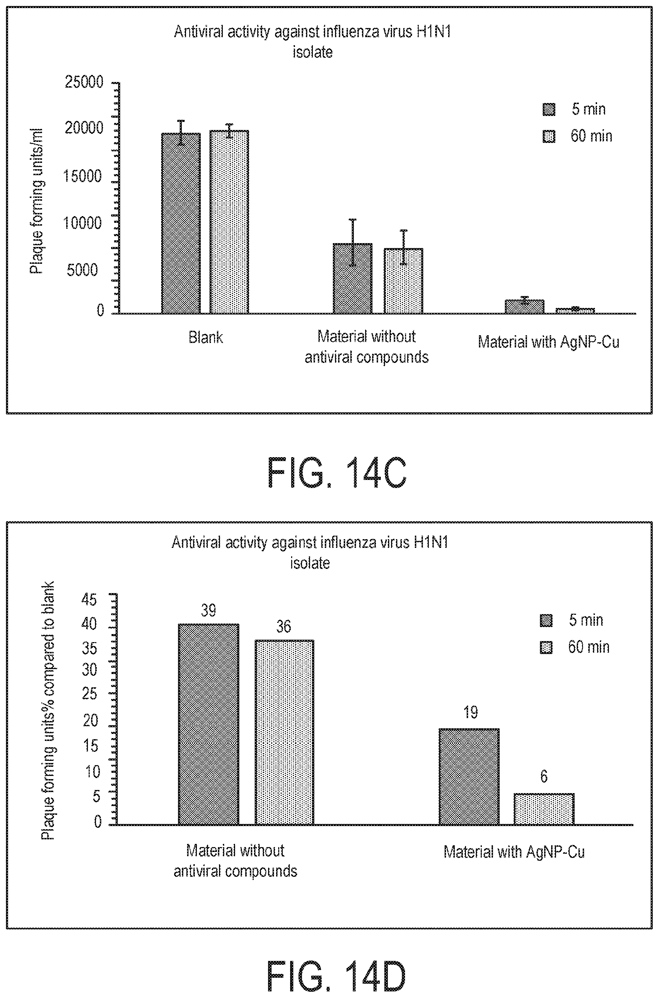

Accordingly, this disclosure provides antiviral material for use for example as face masks or other protective material to reduce or eradicate viral populations. Mask filter material was produced by electrospinning as described in Example 1 with incorporation of 3.2% copper and 0.4% silver nanoparticles. Pore size of skin contact layer was about 200 nm. Materials containing Ag and Cu compounds significantly inactivated SARS-CoV-2 and influenza viruses already after 5 minutes of exposure (Figure 14A and 14C, respectively), whereas after 1 hour the inactivation of the virus was almost complete: 100% in case of SARS-CoV-2 (Figure 14B) and 94% in case of influenza (Figure 14D). Thus, this disclosure provides combination of Ag and Cu compounds as an excellent antiviral agent effective against influenza and SARS-CoV-2. The filter materials intended for the antiviral and antibacterial face masks and produced by electrospinning by incorporating a mixture of Ag and Cu compounds into the silk fibers showed high and fast antiviral effects.

**Example 1.** Preparing antimicrobial material

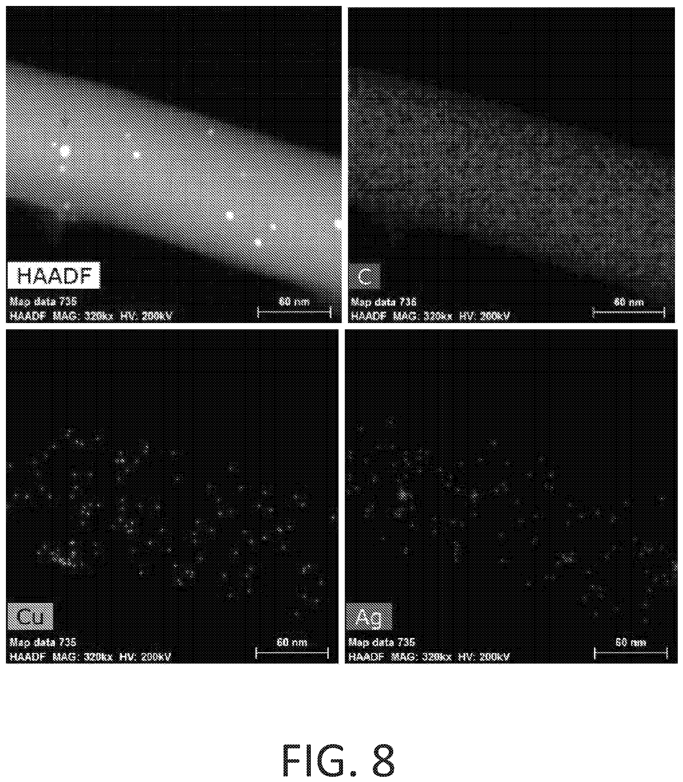

In making a prototype, Nylon 6 was dissolved in formic acid at various concentrations (15-20 wt %) and the solutions were stirred overnight at magnetic heated plate stirrer at 40°C to ensure full dissolution. 0-2000 ppm Cu-AgNP suspensions were stirred with nylon 6-formic acid for 1 h at 40° and then subjected to the electrospinning as described by Javed et al 2. The polymer solutions were electrospun with a fixed mass flow rate of 0.12 mL/h and a voltage of 15 kV for 2 hours. After preparation of the fibers, scanning electron microscopy was used to determine the morphology and the average diameter of the nanofibers (Figure 7, insert). Figure 8 demonstrates that $CuSO_4$ and Ag NP were successfully incorporated into fibers during electrospinning.

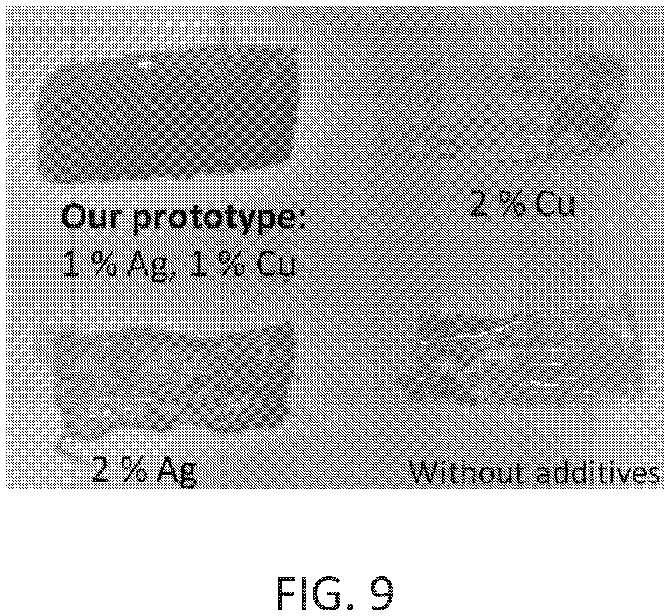

Proposed technology implies that bacteria will be entrapped into the skin contact layer (pore size~200 nm) and will be inactivated on the surface of the wound dressing, where moist wound bed will induce high local release of Cu ions and their interaction with AgNP surface. Figure 9 demonstrates that the wound dressings containing 2% metal from combination of Ag NPs and CuSO4 (1:1) completely inhibited the growth of E. *coli* bacteria, in contrast to the wound dressings that contained 2% metal from Ag NPs or CuSO4 alone.

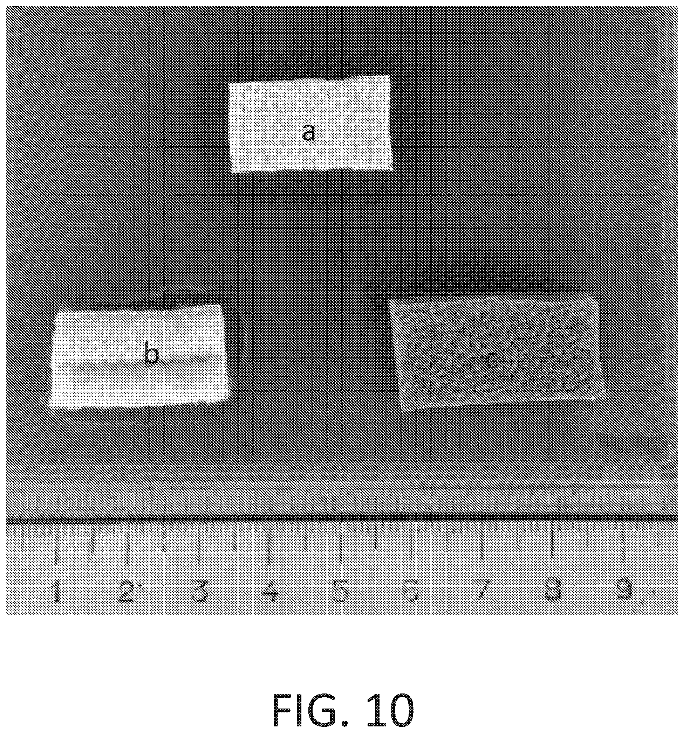

Figure 10 demonstrates that our wound dressing prepared from the optimal Cu-Ag nanoparticle combination inactivates *Escherichia coli* better (larger inhibition zone) (a) compared to commercial Ag-based dressings Aquacel Ag+ by ConvaTec (b) and Mepilex by Mölnlycke (c).

**Example 2** Preparing Cu-alginate gel

A wound dressing comprising Cu ion-based alginate incorporating Ag NPs (and CuO NPs, optionally) can be made as follows: In the first step, Cu-alginate gel will be synthetized from Na-alginate and CuSO4. In the second step, the obtained gel containing Na- and Cu-alginate will be stirred with AgNO3 to obtain *in situ* reduction of Ag ions and formation of Ag NPs or subjected to direct addition of Ag NPs.

In a final (optional) step CuO NPs will be added to the gel. Resulting Cu-alginate gel containing NPs may be used on wounds directly in the form of gel or freeze-dried in several cycles to obtain wound dressing.

**Example 3** Electrospinning nanoparticle containing alginate fibers

Na-alginate can be mixed with Ag NPs and Cu compounds in electrospinning solutions, and NPs-containing alginate fibers will be produced by electrospinning. To enforce the strength of the fibers, additives such as polyethylene glycol or cellulose may be added. In alternative technological solutions for the wound dressings, ion exchange-enabled Cu-alginate incorporating Ag NPs is possible. Upon contact with the wound, Cu-alginate will form a hydrophilic gel releasing NPs and metal ions in the wound environment.

**Example 4** Antibacterial effects of Ag nanoparticles alone and in combination with Cu-compounds

Table 1 demonstrates the raw data (MBCs of Ag NPs and different Cu compounds alone or in combination) used for calculations in Figure 3. Highlighted column demonstrates that antibacterial effect (MBC) can be achieved with remarkably low concentrations of Ag NPs, if used in combination with Cu compounds.

Table 1. Minimal inhibitory concentration (MBC) (mg/l) of Ag NPs and different Cu compounds alone or in combination.

| Bacteria (Gram staining) | MBC alone | MBC(CuX) in combination | MBC(Ag NPs) in combination | K(Syn) |
|---|---|---|---|---|
| *E.coli* K-12 (G-) | | | | |
| Ag NPs | 34,04 $\pm$ 13,1 | | | |

(continued)

| Bacteria (Gram staining) | MBC alone | MBC(CuX) in combination | MBC(Ag NPs) in combination | K(Syn) |
|---|---|---|---|---|
| **E.coli K-12 (G-)** | | | | |
| CuO | 215,4 ± 114,4 | 41,67 ± 14,43 | 7,441 ± 2,577 | 2,387 ± 1,179 |
| CuO-NH$_2$ | 157,1 ± 51,4 | 18,75 ± 7,22 | 5,208 ± 3,348 | 3,047 ± 0,289 |
| CuO-COOH | 350 ± 90,5 | 12,5 ± 0 | 11,90 ± 5,16 | 2,75 ± 0,915 |
| CuSO$_4$ | 217,6 ± 72,8 | 22,5 ± 16,3 | 2,679 ± 0,998 | 5,055 ± 0,383 |
| **E.coli ESBL (G-)** | | | | |
| Ag-col | 45,83 ± 9,73 | | | |
| CuO | 133,3 ± 57,7 | 41,67 ± 14,43 | 7,292 ± 4,774 | 2,222 ± 0,801 |
| CuO-NH$_2$ | 100 ± 0 | 20,83 ± 7,22 | 5,208 ± 1,804 | 3,17 ± 0,536 |
| CuO-COOH | 333,3 ± 115,5 | 58,33 ± 38,19 | 20,83 ± 7,22 | 1,621 ± 0,352 |
| CuSO$_4$ | 200 ± 0 | 25 ± 0 | 4,167 ± 1,804 | 4,727 ± 0,63 |
| **P. aeruginosa PAO1 (G-)** | | | | |
| Ag NPs | 28,91 ± 15,63 | | | |
| CuO | 1400 ± 400 | 43,75 ± 12,5 | 15,63 ± 10,83 | 2,089 ± 0,867 |
| CuO-NH$_2$ | 200 ± 122,5 | 20 ± 6,85 | 8,75 ± 3,423 | 2,324 ± 0,653 |
| CuO-COOH | 800 ± 0 | 34,38 ± 18,75 | 21,88 ± 6,25 | 1,31 ± 0,592 |
| CuSO$_4$ | 720 ± 178,9 | 20 ± 6,85 | 8,75 ± 3,423 | 2,783 ± 0,687 |
| **S.aureus ATCC 25923 (G+)** | | | | |
| Ag NPs | 16,25 ± 5,88 | | | |
| CuO | 60 ± 22,36 | 17,5 ± 6,85 | 2,969 ± 2,096 | 2,18 ± 0,447 |
| CuO-NH$_2$ | 80 ± 27,39 | 12,5 ± 7,65 | 1,875 ± 0,699 | 3,958 ± 0,858 |
| CuO-COOH | 110 ± 54,8 | 11,25 ± 2,8 | 8,125 ± 4,193 | 1,918 ± 1,106 |
| CuSO$_4$ | 90 ± 22,36 | 16,25 ± 8,39 | 1,875 ± 0,699 | 3,659 ± 1,083 |
| **E. faecalis ATCC 29212 (G+)** | | | | |
| Ag NPs | 58,33 ± 19,46 | | | |
| CuO | 233,3 ± 152,8 | 29,17 ± 19,09 | 9,375 ± 5,413 | 3,81 ± 1,374 |
| CuO-NH$_2$ | 266,7 ± 115,5 | 12,5 ± 0 | 10,42 ± 3,61 | 4,655 ± 1,62 |
| CuO-COOH | 400 ± 0 | 16,67 ± 7,22 | 12,5 ± 0 | 3,936 ± 0,983 |
| CuSO$_4$ | 200 ± 0 | 20,83 ± 7,22 | 8,333 ± 3,608 | 4,254 ± 0,977 |
| **S. dysgalacticae DSM 23147 (G+)** | | | | |
| Ag NPs | 10,07 ± 3,76 | | | |
| CuO | 133,3 ± 57,7 | 16,67 ± 7,22 | 1,302 ± 0,451 | 3,766 ± 0,979 |
| CuO-NH$_2$ | 133,3 ± 57,7 | 15,625 ± 13,26 | 3,125 ± 0 | 2,481 ± 0,065 |
| CuO-COOH | 200 ± 0 | 12,5 ± 0 | 6,25 ± 0 | 1,44 ± 0,119 |

(continued)

| S. dysgalacticae DSM 23147 (G+) | | | | |
|---|---|---|---|---|
| $CuSO_4$ | $133{,}3 \pm 57{,}7$ | $12{,}5 \pm 0$ | $1{,}563 \pm 0$ | $3{,}909 \pm 0{,}832$ |

**Example 5** In vivo experiments with rats and clinical testing

Preclinical testing of antibacterial dressing with Wistar rats using the infected wound model was conducted at the Tallinn University of Technology in 2021. The rat's dorsal hair was removed, and the wound site was infiltrated locally with a 1% lidocaine solution to block pain. A round wound about 12 mm in diameter was made using a skin scalpel. 100 $\mu$l of bacterial suspension was placed in the wound. The bacterial suspension was a mixture of 4 bacteria (1: 1: 1: 1): *Escherichia coli* MG1655, *Pseudomonas aeruginosa* PAO1, *Enterococcus faecalis* ATCC 29212 and *Staphylococcus aureus* ATCC 25923. The bacterial concentration was $10^7$/ml. After wound inoculation, the rats were randomly divided into three groups:

- Group 1 - used the wound dressings without active substance (negative control, 9 animals);
- Group 2 - used a commercial antibacterial wound dressing containing the active substance silver - Aquacel Ag+ Extra(positive control, 9 animals);
- Group 3 - used the Advanced Antibacterial Wound Dressing with Ag-col and $CuSO_4$ (test group, 9 animals). Concentration of Ag and Cu in active part of wound dressings were 4% and 8% respectively.

The wound dressing was changed every $2^{nd}$ day. At the same time, the wound was controlled for healing process and also photographed. Wound size, wound edge redness and swelling, wound secretions (blood, transudate, pus) were documented. The general condition of the rat was scored daily.

On days 1, 5, 9, a bacterial inoculum was taken from the wound for bacterial quantitative and qualitative analysis. On days 0, 7 and 21 of the experiment, a blood samples were taken for markers of inflammation (clinical blood, markers of inflammation). On day 21, rats were humanely killed by inhalation of $CO_2$. Blood tests, biopsy at the wound site for histology, microbiology, and biochemical analysis were carried out on the same day. The experiment has shown that the dressing of this disclosure (Advanced Antibacterial Wound Dressing (AAWD) is several orders of magnitude more effective against infection in the wound compared to commercial dressing Aquacel Ag+ Extra (Figure 4). The number of bacteria in the wound has decreased with remarkable speed during the first 24 hours. Bacterial count in wound was 26 times lower than using wound dressings without active substance (negative control group) and 7,6 times lower than using Aquacel Ag+ Extra (group 2). Only *P. aeruginosa* and *S. aureus* have been detected, since they outcompeted other bacterial strains

According to *in vivo* tests, the wound dressing prepared on the basis of Cu-Ag nanoparticle combination according to this invention inactivated pathogenic bacteria *Pseudomonas aeruginosa* 8 times better (3) compared to commercial Ag-based dressing Aquacel Ag+ by ConvaTec (2) (Figure 11)

Cu-Ag nanoparticles based wound dressings are safe and efficient, if applied on patients. They cause less side effects (like allergic reaction, dizziness, nausea, headache compared to competing commercial Ag-based dressing Aquacel Ag+). In addition, Cu-Ag compounds based wound dressings reduce bioburden, inflammation and support the wound healing, when applied on the wounds of patients with diabetic foot ulcer infection, patients with burns and beyond. In addition to the ongoing in vivo tests clinical trials are conducted to prove the safety and efficacy:

*Short description of the clinical trial:*

Study will be performed on 30 patients meeting all of the inclusion criteria and none of the exclusion criteria.

Inclusion Criteria: males and females aged $\geq$18 suffering chronic infection of DFU with a size $\leq$8 cm in diameter and DFU infection grade 2.

Exclusion Criteria: patients receiving antibiotic therapy 7 days before the enrolment, osteomyelitis, cardiac disorder NYHA IV, hepatic disorder Child-Pugh C, cancer stage IV and CLTI, defined as Rutherford's Category $\geq$4 or Fontaine's stage $\geq$III. Patients meeting the eligibility criteria will be enrolled and followed up until day 21. The study performance will be divided into Active Phase (Visit 1 to Visit 3 during 7 days) and Follow-up Phase (Visit 4 to Visit 6 during next 14 days). The patients will be randomly assigned (1:1) to Arm A (AWD) or Arm B (AQUACEL® Ag+). Wound dressings will be changed every second day in the Active Phase. During the Follow-up phase, a non-antibacterial silicon dressing will be used, being changed at least during the study visits and whenever it is required by clinical judgment.

Safety of the AWD will be measured by recording the incidence, the severity and the causal relationship of ADEs, SADEs and DDs, in accordance with the ISO 14155 and the MDCG 2020-10/1 definitions. Safety recording will start after the informed consent has been granted, at the visit 1, and will take until the end of study visit (day 21). All AEs, ADEs, SAEs, SADEs, USADEs and DDs will be recorded.

Efficacy of AWD will be a complex endpoint evaluated through the following variables: microorganisms' load (CFUs) decrease, qualitative microorganisms' changes, inflammatory and infection blood biomarkers' decrease, time until infection resolution (IWGDF grade 1), wound size decrease and wound bed changes.

**Wound appearance** will be assessed at each visit through the following parameters: examination of wound size, edges, bed, debridement: exudates and Wound-related pain (VAS scale). In addition, photographs will be taken at each visit to record the wound healing progress.

The antimicrobial dressing and formulation according to this disclosure provides at least the following benefits:

1) Broad antimicrobial effect. The nanoparticle combination is effective against bacteria (including multi-drug resistant isolates), bacterial biofilms, fungi, protozoa, viruses, and pathological proteins (a-synuclein, prions, amyloid beta). Lower amounts of Ag can be used without compromising the antimicrobial effect.

2) Regenerative function. The nanoparticle combination accelerates the growth of connective tissue cells (fibroblasts, keratinocytes etc.), accelerates wound re-epithelization, helps to revitalize damaged tissues, reduces oxidative stress, restores normal cell structure after damage, reduces the effects of aging, increases tissue concentration of vascular endothelial growth factor (VEGF), platelet-derived growth factor (PDGF), nerve growth factors (NGFs), angiogenin, matrix metalloproteinases (MMP-1, -2, and -9 etc.), copper peptide GHK, fibrinogen which are necessary to restore the tissues.

3) Anti-inflammatory function. Due to the above two points, the nanoparticle combination reduces inflammation in the area of damage and alleviates inflammatory reactions, and thereby reduces further destruction of the tissues. Nanoparticle combination reduces the level of inflammatory factors (such as Tumor Necrosis Factor-$\alpha$, inflammatory interleukins, cytokines, and cyclooxygenases).

4) Anesthetic function. Due to the above properties, these nanoparticle combination alleviate pain from the wound site.

The antimicrobial effects of materials made according to this invention have proven not only to be efficient as wound dressings but also in other antimicrobial uses, including antiviral use:

**Example 6** Testing antiviral effects of the antimicrobial material

Mask filter material was produced as described in Example 1 with incorporation of 3.2% copper and 0.4% silver nanoparticles. The material was tested for antiviral activity against influenza virus and coronavirus SARS-CoV-2.

1) Testing with SARS-CoV-2

Three independent experiments have been performed. Autoclaved 2x2 cm material pieces were placed into 50 ml falcon tubes using single-use forceps. 30 $\mu$l of SARS-CoV-2 virus stock (recombinant, Delta isolate, titer 5,68x $10^5$ pfu/ml) was added to each material piece and incubated for 0 h and 1 hour at 28°C. After incubation, 470 $\mu$l of 1x PBS (phosphate-buffered saline) was added to each tube after a brief vortexing the tubes were centrifuged at 3200 x g for 3 min at room temperature. The supernatants were transferred into the 1.5 ml reaction tubes. Serial 10-fold dilutions of the supernatants (starting from $10^0$ to $10^{-3}$) were prepared in a U-bottom 96 well plate using Virus Growth Medium (VGM - DMEM, 0.2% BSA, 100 IU/ml Penicillin, 100 $\mu$g/ml Streptomycin), and Vero E6 cells pre-seeded on the flat-bottom 96-well plate were infected with 100 $\mu$l of dilutions for 1 hour at 37°C. Next, infected cells were overlaid with VGM/1% CMC (carboxymethyl cellulose) and incubated in a humidified atmosphere at 37°C and 5% $CO_2$. After 48 hours, infected cells were fixed with ice-cold 80% acetone/PBS for 1 hour at - 20°C, and the plate was dried under the hood overnight. Next, an immunoplaque assay was performed to count the microplaques. Cells treated with supernatants from materials without antimicrobial compounds were used as negative controls. *Immunoplaque assay*

96-well plate fixed with acetone was blocked using 1x Pierce Clear Milk blocking buffer (Thermo Scientific, Cat. Nr. 37587) for 1 hour at 37°C. Next, the plate was probed using anti-SARS-CoV2-N monoclonal rabbit antibody 82C3 (Icosagen AS, Tartu, Estonia) at a dilution 1:5000 in 1x Pierce Clear Milk blocking buffer for 1 hour at 37°C, and secondary anti-rabbit goat IRDyeCW800-conjugated antibody (LI-COR Biosciences, NE, USA) diluted 1:5000 in 1x Pierce Clear Milk blocking buffer for 1 hour at 37°C. The plate was then dried under the hood for 30 minutes, and the fluorescent signal was read using LI-COR scanning machine (wavelength 800 nm). The number of fluorescent plaques was counted in the appropriate dilution wells (5-50 plaques per well), the avarage of the three experiments was calculated and compared to the viral titers in the wells treated with negative controls.

2) Testing with influenza virus

Three independent experiments have been performed. 2x2 cm autoclaved materials were placed into the wells of 12-well plates. 30 $\mu$l of influenza virus (A/WSN/1933 (H1N1)) stock (6*10$^5$ pfu/ml, 1,8*10$^4$ pfu-s in 30 $\mu$l) was added to materials and then 470 $\mu$l pure DMEM medium was added per well (final volume of the sample was 500 $\mu$l). Materials were incubated with the virus for 0h or 1h. 1h incubation was carried out at 28°C, 5 % $CO_2$ in a humidified atmosphere. To collect the sample, the liquid from the well was transferred into 1,5 ml tube. 10 times serial dilutions were prepared from each sample in pure DMEM and these dilutions were used to infect 100% confluent Madin-Darby canine kidney (MDCK-2) cells on 12-well plates to titrate the samples by plaque assay. Cells were infected in 150 $\mu$l per well for 1h at 37°C, 5 % $CO_2$, humidified atmosphere, gently shaking the plates. After 1h the medium was replaced with 3:2 virus growth medium:2 % carboxymethylcellulose (~1,5 ml/well) containing N-tosyl-L-phenylalanine chloromethyl ketone (TPCK) trypsin (final concentration 1 $\mu$g/ml) and plates were incubated for 96h until the plaques formed. 96 h post-infection the medium was removed and cells were dyed using crystal violet. Plates were washed, plaques were counted, virus titers of the samples were calculated and compared to control titers.

Materials containing Ag and Cu compounds significantly inactivated SARS-CoV-2 and influenza viruses already after 5 minutes of exposure (Figure 14A and 14C, respectively), whereas after 1 hour the inactivation of the virus was almost complete: 100% in case of SARS-CoV-2 (Figure 14B) and 94% in case of influenza (Figure 14D). The experiments show that a combination of Ag and Cu compounds according to this disclosure is an excellent antiviral agent effective against influenza and SARS-CoV-2. The filter materials intended for the antimicrobial face masks and produced by electrospinning by incorporating a mixture of Ag and Cu compounds into the silk fibers showed high and fast antiviral effects.

This combination can be incorporated into various polymers, textiles and materials including but not limited to non-woven as well woven materials, various biodegradable materials (alginate, cellulose (e.g., cellulose ethyl sulphonate, carboxymethylcellulose), collagen, chitin, silk, cotton, polylactic acid), nylon, polyethylene, silicone, polyvinyl alcohol, polyacrylonitrile, polyaniline, polyvinylpyrrolidone, polypropylene, polyurethane, paraffin, viscose, polyester, styrene-acrylonitrile and others using various methods including but not limited to electrospinning, infusion, adsorption, absorption, melt blowing, sonochemistry, plating, impregnation, or electrodeposition etc. techniques to give to these materials excellent antiviral properties.

## Claims

1. A medical device comprising a silver component and a copper component,

   wherein the combination of silver and copper components provides a synergistic antimicrobial effect,
   wherein the silver component comprises Ag nanoparticles alone or in combination with ionic silver, and the copper component comprises Cu-based salts selected from $CuSO_4$.

2. The medical device according to claim 1, wherein the silver and copper components are in such amounts that Cu/Ag ratio is between 1 to 70, more preferably between 1 to 20, most preferably between 1 to 8,

   preferably wherein the device is an antimicrobial dressing,
   more preferably wherein the device is an antimicrobial dressing and Ag nanoparticles are either non functionalized or functionalized with one or more groups selected from carboxyl functional groups (-COOH), quaternary ammonium, polyethylene imine, branched polyethylene imine, PEG, citrate, PVP, dextran coating, amine groups (-NH$_2$), amino acids, bacteria binding peptides, proteins, polyoxometalates (POMs), antibodies, their fragments, and combinations thereof.

3. The medical device according to claim 2, wherein the device is antimicrobial dressing, and the Ag nanoparticles are protein stabilized,
   preferably wherein the device is antimicrobial dressing, and the silver component comprises Ag nanoparticles and Cu component comprises Cu salt selected from $CuSO_4$, and the Ag and Cu components are embedded in fibers, preferably by electrospinning.

4. The medical device of claim 3, wherein the device is antimicrobial dressing and coefficient of antimicrobial synergy when measured as

$$K(Syn) = 1 / \left( \frac{MBC \text{ of Ag comp in mix}}{MBC \text{ of Ag comp alone}} + \frac{MBC \text{ of Cu comp in mix}}{MBC \text{ of Cu comp alone}} \right)$$

is higher than 1, preferably higher than 2, more preferably higher than 3, and most preferably higher than 4.

5. The medical device of claim 4, wherein the device is antimicrobial dressing and wherein applying the dressing on a wound improves the wound infection and healing outcome in comparison to applying a dressing with only the Ag compound or only the Cu compound.

6. The medical device according to claim 4, wherein the device is antimicrobial dressing made of foam, film, proteins, polysaccharides, hydrogel, hydrocolloid, or hydrofibers by using electrospinning or mixing and the Ag and Cu components are applied on the materials via sonochemistry, plating, impregnation, electrospinning, adsorption, melt blowing or electrodeposition techniques, preferably wherein the device is antimicrobial dressing and materials of the dressing are biopolymers, such as alginate, cellulose (e.g., cellulose ethyl sulphonate, carboxymethylcellulose), collagen, bamboo, fibronectin, chitin, silk fibroin, cotton, or synthetic polymers such as nylon, polyethylene, silicone, polyvinyl alcohol, polyacrylonitrile, polyaniline, polyvinylpyrrolidone, polypropylene, polyurethane, viscose, paraffin, polyester or other plastics.

7. The medical device according to claim 6, wherein the device is antimicrobial dressing having a skin contact layer comprising the Ag and Cu components, and optionally an absorbant layer comprising a polymer, such as polyethylene or collagen bamboo, fibronectin, chitin, silk fibroin, cotton, or synthetic polymers such as nylon, polyethylene, silicone, polyvinyl alcohol, polyacrylonitrile, polyaniline, polyvinylpyrrolidone, polypropylene, polyurethane, viscose, paraffin, polyester or other plastics,
preferably wherein the device is an antimicrobial dressing, and the skin contact layer is silicone, silk fibroin, collagen or paraffin net comprising the Ag- and the Cu-compounds.

8. An antimicrobial formulation comprising silver and copper components, wherein the silver component includes Ag nanoparticles alone or in combination with ionic Ag, and Cu component include Cu-based salts selected from $CuSO_4$.

9. The antimicrobial formulation according to claim 8 comprising silver and copper components in such proportions that Cu/Ag ratio is between 1 to 70, more preferably between 1 to 20, most preferably between 1 and 8.

10. The antimicrobial formulation according to claim 9, wherein Ag nanoparticles are either non-functionalized or functionalized with one or more groups selected from carboxyl functional groups (-COOH), quaternary ammonium, polyethylene imine, branched polyethylene imine, PEG, citrate, PVP, dextran coating, amine groups ($-NH_2$), amino acids, bacteria binding peptides, polyoxometalates (POMs), proteins, antibodies, their fragments, and combinations thereof.

11. The antimicrobial formulation according to claim 9, wherein a coefficient of antimicrobial synergy of the formulation when measured as

$$K(Syn) = 1 / \left( \frac{MBC \text{ of Ag compounds in mix}}{MBC \text{ of Ag compounds alone}} + \frac{MBC \text{ of Cu compounds in mix}}{MBC \text{ of Cu/-compounds alone}} \right)$$

is higher than 1, preferably higher than 2, more preferably higher than 3, and most preferably higher than 4.

12. The antimicrobial formulation according to claim 9 for use in preventing or diminishing microbial growth on human tissue or on surfaces of a medical device.

13. The antimicrobial formulation for use according to claim 12, wherein the formulation is applied directly or indirectly via a medical device onto human tissue.

14. The medical device of claim 4 or the formulation of claim 11 for use in treating a wound or an inflamed tissue area.

15. The dressing according to any of claims 3 to 7 for use in a method to improve healing of a wound, especially a diabetic ulcer, the method comprising administering the dressing on the wound wherein the improvement is measured as faster removal of antibacterial infection and hence, faster healing time.

**Patentansprüche**

1. Medizinische Vorrichtung, die eine Silberkomponente und eine Kupferkomponente umfasst,

   wobei die Kombination aus Silber- und Kupferkomponenten eine synergistische antimikrobielle Wirkung bereitstellt,
   wobei die Silberkomponente Ag-Nanopartikel allein oder in Kombination mit ionischem Silber umfasst und die Kupferkomponente Cu-basierte Salze umfasst, ausgewählt aus $CuSO_4$.

2. Medizinische Vorrichtung nach Anspruch 1, wobei die Silber- und Kupferkomponenten in solchen Mengen vorhanden sind, dass das Cu/Ag-Verhältnis zwischen 1 und 70, mehr bevorzugt zwischen 1 und 20, am meisten bevorzugt zwischen 1 und 8 liegt,

   wobei es sich bei der Vorrichtung vorzugsweise um einen antimikrobiellen Verband handelt,
   wobei es sich bei der Vorrichtung mehr bevorzugt um einen antimikrobiellen Verband handelt und die Ag-Nanopartikel entweder nicht funktionalisiert oder mit einer oder mehreren Gruppen funktionalisiert sind, ausgewählt aus funktionellen Carboxylgruppen (-COOH), quartärem Ammonium, Polyethylenimin, verzweigtem Polyethylenimin, PEG, Citrat, PVP, Dextranbeschichtung, Amingruppen ($-NH_2$), Aminosäuren, bakterienbindenden Peptiden, Proteinen, Polyoxometallaten (POMs), Antikörpern, deren Fragmenten und Kombinationen davon.

3. Medizinische Vorrichtung nach Anspruch 2, wobei es sich bei der Vorrichtung um einen antimikrobiellen Verband handelt und die Ag-Nanopartikel proteinstabilisiert sind,
   wobei es sich bei der Vorrichtung vorzugsweise um einen antimikrobiellen Verband handelt und die Silberkomponente Ag-Nanopartikel umfasst und die Cu-Komponente Cu-Salz umfasst, ausgewählt aus $CuSO_4$, und die Ag- und Cu-Komponenten in Fasern eingebettet werden, vorzugsweise durch Elektrospinnen.

4. Medizinische Vorrichtung nach Anspruch 3, wobei die Vorrichtung ein antimikrobieller Verband ist und der Koeffizient der antimikrobiellen Synergie, wenn er wie folgt gemessen wird,

$$K(Syn) = 1/ \left( \frac{\text{MBK von Ag-Verbindungen, gemischt}}{\text{MBK von Ag-Verbindungen, allein}} + \frac{\text{MBK von Cu-Verbindungen, gemischt}}{\text{MBK von Cu-Verbindungen, allein}} \right)$$

   höher als 1, vorzugsweise höher als 2, mehr bevorzugt höher als 3 und am meisten bevorzugt höher als 4 ist.

5. Medizinische Vorrichtung nach Anspruch 4, wobei die Vorrichtung ein antimikrobieller Verband ist und wobei das Aufbringen des Verbands auf eine Wunde die Wundinfektion und das Outcome der Heilung im Vergleich zum Aufbringen eines Verbands mit nur der Ag-Verbindung oder nur der Cu-Verbindung verbessert.

6. Medizinische Vorrichtung nach Anspruch 4, wobei die Vorrichtung ein antimikrobieller Verband ist, der aus Schaumstoff, Folie, Proteinen, Polysacchariden, Hydrogel, Hydrokolloid oder Hydrofasern unter Anwendung von Elektrospinnen oder Mischen hergestellt wird, und wobei die Ag- und Cu-Komponenten mittels Sonochemie, Plattieren, Imprägnieren, Elektrospinnen, Adsorption, Schmelzblasen oder Elektroabscheidungstechniken auf die Materialien aufgetragen werden, wobei die Vorrichtung vorzugsweise ein antimikrobieller Verband ist und die Materialien des Verbands Biopolymere sind, wie etwa Alginat, Zellulose (z. B. Zelluloseethylsulfonat, Carboxymethylzellulose), Kollagen, Bambus, Fibronektin, Chitin, Seidenfibroin, Baumwolle oder synthetische Polymere, wie etwa Nylon, Polyethylen, Silikon, Polyvinylalkohol, Polyacrylnitril, Polyanilin, Polyvinylpyrrolidon, Polypropylen, Polyurethan, Viskose, Paraffin, Polyester oder andere Kunststoffe.

7. Medizinische Vorrichtung nach Anspruch 6, wobei die Vorrichtung ein antimikrobieller Verband mit einer Hautkontaktschicht ist, die die Ag- und Cu-Komponenten umfasst, und optional eine absorbierende Schicht, die ein Polymer wie Polyethylen oder Kollagen, Bambus, Fibronektin, Chitin, Seidenfibroin, Baumwolle oder synthetische Polymere wie Nylon, Polyethylen, Silikon, Polyvinylalkohol, Polyacrylnitril, Polyanilin, Polyvinylpyrrolidon, Polypropylen, Polyurethan, Viskose, Paraffin, Polyester oder andere Kunststoffe umfasst,

wobei es sich bei der Vorrichtung vorzugsweise um einen antimikrobiellen Verband handelt und die Hautkontaktschicht aus Silikon, Seidenfibroin, Kollagen oder einem Paraffinnetz besteht, umfassend die Ag- und die Cu-Verbindungen.

8. Antimikrobielle Formulierung, die Silber- und Kupferkomponenten umfasst, wobei die Silberkomponente Ag-Nanopartikel allein oder in Kombination mit ionischem Ag einschließt und die Cu-Komponente Cu-basierte Salze einschließt, ausgewählt aus $CuSO_4$.

9. Antimikrobielle Formulierung nach Anspruch 8, die Silber- und Kupferkomponenten in solchen Anteilen umfasst, dass das Cu/Ag-Verhältnis zwischen 1 und 70, mehr bevorzugt zwischen 1 und 20 und am meisten bevorzugt zwischen 1 und 8 liegt.

10. Antimikrobielle Formulierung nach Anspruch 9, wobei Ag-Nanopartikel entweder nicht funktionalisiert oder mit einer oder mehreren Gruppen funktionalisiert sind, ausgewählt aus funktionellen Carboxylgruppen (-COOH), quartärem Ammonium, Polyethylenimin, verzweigtem Polyethylenimin, PEG, Citrat, PVP, Dextranbeschichtung, Amingruppen ($-NH_2$), Aminosäuren, bakterienbindenden Peptiden, Polyoxometallaten (POMs), Proteinen, Antikörpern, deren Fragmenten und Kombinationen davon.

11. Antimikrobielle Formulierung nach Anspruch 9, wobei ein Koeffizient der antimikrobiellen Synergie der Formulierung, wenn er wie folgt gemessen wird,

$$K(Syn) = 1/ \left( \frac{\text{MBK von Ag-Verbindungen, gemischt}}{\text{MBK von Ag-Verbindungen, allein}} + \frac{\text{MBK von Cu-Verbindungen, gemischt}}{\text{MBK von Cu-Verbindungen, allein}} \right)$$

höher als 1, vorzugsweise höher als 2, mehr bevorzugt höher als 3 und am meisten bevorzugt höher als 4 ist.

12. Antimikrobielle Formulierung nach Anspruch 9 zur Verwendung bei der Verhinderung oder Verringerung von mikrobiellem Wachstum auf menschlichem Gewebe oder auf Oberflächen einer medizinischen Vorrichtung.

13. Antimikrobielle Formulierung zur Verwendung nach Anspruch 12, wobei die Formulierung direkt oder indirekt über eine medizinische Vorrichtung auf menschliches Gewebe aufgetragen wird.

14. Medizinische Vorrichtung nach Anspruch 4 oder Formulierung nach Anspruch 11 zur Verwendung bei der Behandlung einer Wunde oder eines entzündeten Gewebebereichs.

15. Verband nach einem der Ansprüche 3 bis 7 zur Verwendung in einem Verfahren zur Verbesserung der Heilung einer Wunde, insbesondere eines diabetischen Ulkus, wobei das Verfahren das Aufbringen des Verbands auf die Wunde umfasst, wobei die Verbesserung als schnellere Entfernung der antibakteriellen Infektion und damit als schnellere Heilungszeit gemessen wird.


**Revendications**

1. Dispositif médical comprenant un composant d'argent et un composant de cuivre,

dans lequel la combinaison de composants d'argent et de cuivre produit un effet antimicrobien synergique, dans lequel le composant d'argent comprend des nanoparticules d'Ag seules ou en combinaison avec de l'argent ionique, et le composant de cuivre comprend des sels à base de Cu choisis parmi $CuSO_4$.

2. Dispositif médical selon la revendication 1, dans lequel les composants d'argent et de cuivre sont en quantités telles que le rapport Cu/Ag est compris entre 1 et 70, plus préférablement entre 1 et 20, le plus préférablement encore entre 1 et 8,

de préférence dans lequel le dispositif est un pansement antimicrobien,
plus préférablement dans lequel le dispositif est un pansement antimicrobien et les nanoparticules d'Ag sont soit non fonctionnalisées, soit fonctionnalisées avec un ou plusieurs groupes choisis parmi des groupes fonctionnels carboxyle (-COOH), l'ammonium quaternaire, la polyéthylèneimine, la polyéthylèneimine ramifiée, le PEG, le citrate, le PVP, un revêtement de dextran, des groupes amine (-NH$_2$), des acides aminés, des peptides liant les bactéries, des protéines, des polyoxométalates (POM), des anticorps, leurs fragments, et des combinaisons de ceux-ci.

3. Dispositif médical selon la revendication 2, dans lequel le dispositif est un pansement antimicrobien, et les nanoparticules d'Ag sont stabilisées par des protéines, de préférence dans lequel le dispositif est un pansement antimicrobien, et le composant d'argent comprend des nanoparticules d'Ag et le composant de Cu comprend un sel de Cu choisi parmi CuSO$_4$, et les composants d'Ag et de Cu sont incorporés dans des fibres, de préférence par filage électrostatique.

4. Dispositif médical selon la revendication 3, dans lequel le dispositif est un pansement antimicrobien et le coefficient de synergie antimicrobienne lorsque mesuré comme suit

$$K(\mathbf{Syn}) = 1/ \left( \frac{\text{CBM du comp d'Ag dans le mélange}}{\text{CBM du comp d'Ag seul}} + \frac{\text{CBM du comp de Cu dans le mélange}}{\text{CBM du comp de Cu seul}} \right)$$

est supérieur à 1, de préférence supérieur à 2, plus préférablement supérieur à 3, et le plus préférablement supérieur à 4.

5. Dispositif médical selon la revendication 4, dans lequel le dispositif est un pansement antimicrobien et dans lequel l'application du pansement sur une plaie améliore l'infection de la plaie et la cicatrisation par rapport à l'application d'un pansement contenant uniquement le composé d'Ag ou uniquement le composé de Cu.

6. Dispositif médical selon la revendication 4, dans lequel le dispositif est un pansement antimicrobien fait de mousse, de film, de protéines, de polysaccharides, d'hydrogel, d'hydrocolloïde ou d'hydrofibres en utilisant le filage électrostatique ou le mélange et les composants d'Ag et de Cu sont appliqués sur les matériaux par sonochimie, placage, imprégnation, filage électrostatique, adsorption, fusion-soufflage ou techniques d'électrodéposition, de préférence dans lequel le dispositif est un pansement antimicrobien et les matériaux du pansement sont des biopolymères, tels que l'alginate, la cellulose (p. ex., le sulfonate d'éthyle de cellulose, la carboxyméthylcellulose), le collagène, le bambou, la fibronectine, la chitine, la fibroïne de soie, le coton, ou des polymères synthétiques tels que le nylon, le polyéthylène, la silicone, l'alcool polyvinylique, le polyacrylonitrile, la polyaniline, le polyvinylpyrrolidone, le polypropylène, le polyuréthane, la viscose, la paraffine, le polyester ou d'autres matières plastiques.

7. Dispositif médical selon la revendication 6, dans lequel le dispositif est un pansement antimicrobien ayant une couche de contact avec la peau comprenant les composants d'Ag et de Cu, et éventuellement une couche absorbante comprenant un polymère, tel que le polyéthylène ou le collagène, le bambou, la fibronectine, la chitine, la fibroïne de soie, le coton, ou des polymères synthétiques tels que le nylon, le polyéthylène, la silicone, l'alcool polyvinylique, le polyacrylonitrile, la polyaniline, le polyvinylpyrrolidone, le polypropylène, le polyuréthane, la viscose, la paraffine, le polyester ou d'autres matières plastiques, de préférence dans lequel le dispositif est un pansement antimicrobien et la couche de contact avec la peau est un filet de silicone, de fibroïne de soie, de collagène ou de paraffine comprenant les composés d'Ag et de Cu.

8. Formulation antimicrobienne comprenant des composants d'argent et de cuivre, dans laquelle le composant d'argent comporte des nanoparticules d'Ag seules ou en combinaison avec de l'Ag ionique, et le composant de Cu comporte des sels à base de Cu choisis parmi CuSO$_4$.

9. Formulation antimicrobienne selon la revendication 8, comprenant des composants d'argent et de cuivre dans des proportions telles que le rapport Cu/Ag est compris entre 1 et 70, plus préférablement entre 1 et 20, le plus préférablement entre 1 et 8.

10. Formulation antimicrobienne selon la revendication 9, dans laquelle les nanoparticules d'Ag sont soit non fonctionnalisées, soit fonctionnalisées avec un ou plusieurs groupes choisis parmi des groupes fonctionnels carboxyle (-COOH), l'ammonium quaternaire, la polyéthylèneimine, la polyéthylèneimine ramifiée, le PEG, le citrate, le PVP, un revêtement de dextran, des groupes amine (-NH$_2$), des acides aminés, des peptides liant les bactéries, des polyoxométalates (POM), des protéines, des anticorps, leurs fragments et des combinaisons de ceux-ci.

11. Formulation antimicrobienne selon la revendication 9, dans laquelle un coefficient de synergie antimicrobienne de la formulation lorsque mesuré comme suit

$$K(\text{Syn}) = 1/ \left( \frac{\text{CBM des composés d'Ag dans le mélange}}{\text{CBM des composés d'Ag seuls}} + \frac{\text{CBM des composés de Cu dans le mélange}}{\text{CBM des composés de Cu seuls}} \right)$$

est supérieur à 1, de préférence supérieur à 2, plus préférablement supérieur à 3, et le plus préférablement supérieur à 4.

12. Formulation antimicrobienne selon la revendication 9 pour utilisation dans la prévention ou la diminution de la croissance microbienne sur un tissu humain ou sur des surfaces d'un dispositif médical.

13. Formulation antimicrobienne pour une utilisation selon la revendication 12, dans laquelle la formulation est appliquée directement ou indirectement sur un tissu humain par l'intermédiaire d'un dispositif médical.

14. Dispositif médical selon la revendication 4 ou formulation selon la revendication 11 pour une utilisation dans le traitement d'une plaie ou d'une zone tissulaire enflammée.

15. Pansement selon l'une quelconque des revendications 3 à 7 pour une utilisation dans un procédé visant à améliorer la cicatrisation d'une plaie, en particulier un ulcère diabétique, le procédé comprenant l'administration du pansement sur la plaie, dans lequel l'amélioration est mesurée par une élimination plus rapide de l'infection antibactérienne et, par conséquent, par un temps de cicatrisation plus court.

# FIG. 1

# FIG. 2

FIG. 3B

FIG. 3A

FIG. 4

FIG. 5

FIG. 6A

FIG. 6B

Nanofibers
With antibacterial
nanoparticles

Skin contact
nanofiber layer

- Kills bacteria
- Reduces inflammation
- Improves wound healing

Absorbent layer

- Advanced polymer
- Absorbs wound exudate

# FIG. 7

FIG. 8

FIG. 9

FIG. 10

FIG. 11A

FIG. 11B

FIG. 12

FIG. 13

FIG. 14A

FIG. 14B

FIG. 14C

FIG. 14D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6471982 B1
- WO 2003053484 A
- US 20030021832 A
- US 8058499 B2
- US 20030176827 A1
- EP 1901723 A2
- US 9838652 B
- US 20160022606 A
- WO 2012162557 A

### Non-patent literature cited in the description

- **CHUN-CHI LIANG** ; **ANN Y PARK** ; **JUN-LIN GUAN**. In vitro scratch assay: a convenient and inexpensive method for analysis of cell migration in vitro. *Nature Protocols*, 2007, vol. 2, 329-333